(19) Europäisches Patentamt
European Patent Office
Office européen des brevets

(11) Numéro de publication : **0 555 153 A1**

(12) **DEMANDE DE BREVET EUROPEEN**

(21) Numéro de dépôt : **93400289.0**

(51) Int. Cl.$^5$ : **C07D 307/42,** A01N 53/00

(22) Date de dépôt : **05.02.93**

(30) Priorité : **07.02.92 FR 9201391**

(43) Date de publication de la demande :
**11.08.93 Bulletin 93/32**

(84) Etats contractants désignés :
**CH DE FR GB IT LI NL**

(71) Demandeur : **ROUSSEL-UCLAF**
**35, Boulevard des Invalides**
**F-75007 Paris (FR)**

(72) Inventeur : **Benoit, Marc**
**Le Cannet Est, Pont de l'Etoile**
**F-13360 Roquevaire (FR)**
Inventeur : **Demassey, Jacques**
**Allée Saint Jacques Chalifert**
**F-77144 Montevrain (FR)**
Inventeur : **Demoute, Jean-Pierre**
**65, Avenue Foch**
**F-93360 Neuilly Plaisance (FR)**

(74) Mandataire : **Tonnellier, Marie-José et al**
**111, route de Noisy B.P. no 9**
**F-93230 Romainville (FR)**

(54) **Nouveaux esters pyréthrinoides dérivés d'alcool furanique ou thiophénique, leur procédé de préparation et leur application comme pesticides.**

(57) L'invention a pour objet les composés de formule (I) :

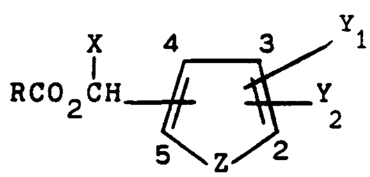

(I)

dans lesquels,
— le radical

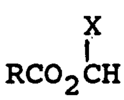

est en position 2 ou 3,
— X représente un atome d'hydrogène, un radical alkyle linéaire ou ramifié renfermant jusqu'à 8 atomes de carbone, saturé ou insaturé, ou un radical C≡N,
— $Y_1$ et $Y_2$, identiques ou différents en position 2, 3, 4 ou 5 représentent un atome d'hydrogène, un atome d'halogène, un radical

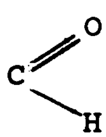

,

$NO_2$, $NH_2$ ou C-=N, un radical alkyle ou O-alkyle, ou

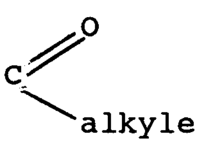

ou

EP 0 555 153 A1

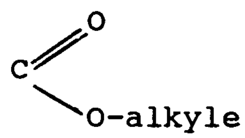

linéaire, ramifié ou cyclique, saturé ou insaturé renfermant jusqu'à 8 atomes de carbone éventuellement substitué par un ou plusieurs atomes d'halogène, un radical $-(CH_2)_nOH$, n représentant un nombre entier égal à 0, 1, 2, 3 ou 4 dont le radical OH peut être éventuellement éthérifié ou estérifié, ou un radical

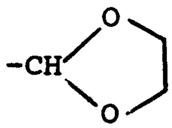

— Z représente un atome d'oxygène ou de soufre.
Les composés de formule (I) présentent d'intéressantes propriétés pesticides.

La présente invention concerne de nouveaux esters pyréthrinoïdes dérivés d'alcool furanique ou thiophénique, leur procédé de préparation et leur application comme pesticides.

L'invention a pour objet sous toutes leurs formes stéréoisomères possibles ainsi que les mélanges de ces stéréoisomères, les composés de formule (I) :

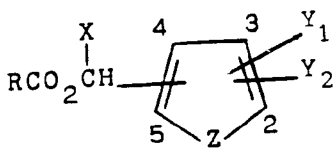

(I)

dans lesquels,

- le radical

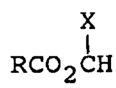

  est en position 2 ou 3,
- X représente un atome d'hydrogène, un radical alkyle linéaire ou ramifié renfermant jusqu'à 8 atomes de carbone, saturé ou insaturé, ou un radical C≡N,
- $Y_1$ et $Y_2$, identiques ou différents en position 2, 3, 4 ou 5 représentent un atome d'hydrogène, un atome d'halogène, un radical

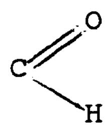

,

$NO_2$, $NH_2$ ou C≡N, un radical alkyle ou O-alkyle, ou

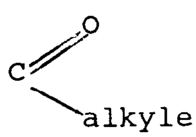

ou

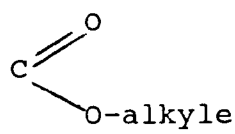

linéaire, ramifié ou cyclique, saturé ou insaturé renfermant jusqu'à 8 atomes de carbone éventuellement substitué par un ou plusieurs atomes d'halogène, un radical $-(CH_2)_nOH$, n représentant un nombre entier égal à 0, 1, 2, 3 ou 4 dont le radical OH peut être éventuellement éthérifié ou estérifié, ou un radical

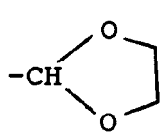

- Z représente un atome d'oxygène ou de soufre,
  et ou bien R représente un radical :

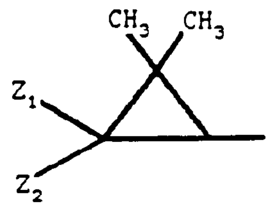

dans lequel :

- $Z_1$ et $Z_2$ représentent chacun un radical méthyle,
- ou $Z_1$ représente un atome d'hydrogène et
- soit $Z_2$ représente un radical :

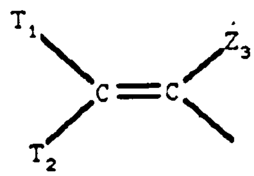

dans lequel $Z_3$ représente un atome d'hydrogène ou d'halogène et $T_1$ et $T_2$, identiques ou différents, représentent un atome d'hydrogène, un atome d'halogène, un radical alkyloxy ou alkyle renfermant de 1 à 8 atomes de carbone éventuellement substitués par des halogènes, un radical mono-, di- ou trifluorométhyle ou cyano ou un noyau phényle éventuellement substitué par un halogène, ou $T_1$ et $T_2$ forment ensemble un radical cycloalkyle renfermant de 3 à 6 atomes de carbone ou un radical :

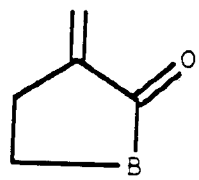

dans lequel B représente un atome d'oxygène ou de soufre ;

- soit $Z_2$ représente un radical :

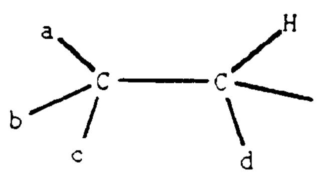

dans lequel a, b, c et d, identiques ou différents représentent chacun un atome d'halogène,

- soit $Z_2$ représente un radical :

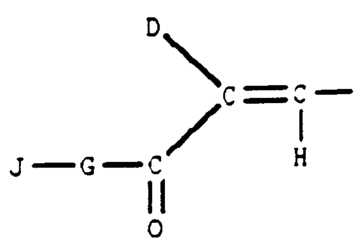

dans lequel D représente un atome d'hydrogène ou d'halogène, un radical alkyloxy renfermant de 1 à 8 atomes de carbone, G représente un atome d'oxygène ou de soufre et J représente un radical alkyle linéaire, ramifié ou cyclique, saturé ou insaturé, renfermant de 1 à 8 atomes de carbone, éventuellement

substitué par un ou plusieurs groupements fonctionnels, identiques ou différents, ou bien un groupement aryle renfermant de 6 à 14 atomes de carbone, éventuellement substitué par un ou plusieurs groupements fonctionnels identiques ou différents, ou bien un radical hétérocyclique éventuellement substitué par un ou plusieurs groupements fonctionnels, identiques ou différents,
ou bien R représente un radical :

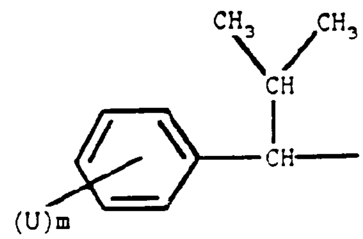

dans lequel U, en position quelconque sur le noyau benzénique, représente un atome d'halogène, un radical alkyle renfermant de 1 à 8 atomes de carbone ou un radical alcoxy renfermant de 1 à 8 atomes de carbone, m représentant le nombre 0, 1 ou 2 et quand m est 2, les substituants U peuvent être identiques ou différents.

Lorsque $Y_1$ ou $Y_2$ représente un atome d'halogène, il s'agit de préférence d'un atome de fluor, de chlore ou de brome.

Lorsque X représente un radical alkyle saturé ou insaturé, il s'agit de préférence d'un radical méthyle, éthyle ou éthynyle.

Lorsque $Y_1$ ou $Y_2$ représente un radical alkyle, il s'agit de préférence d'un radical méthyle, éthyle, propyle, isopropyle, n-butyle, isobutyle, terbutyle, vinyle, allyle, éthynyle ou propynyle.

Lorsque $Y_1$ ou $Y_2$ est substitué par un atome d'halogène, l'halogène est de préférence un atome de fluor ou de chlore, il peut s'agir par exemple du radical $CF_3$, $CHF_3$, $CHCl_2$ ou $CH_2F$, ou encore

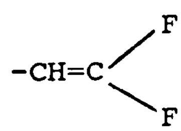

Lorsque $Y_1$ ou $Y_2$ représente un radical O-alkyle, alkyle a de préférence l'une des valeurs indiquées ci-dessus.

Lorsque Y représente un radical $(CH_2)_nOH$, libre éthérifié ou estérifié, il s'agit de préférence du radical $CH_2OH$, $CH_2OCH_3$ ou $CH_2OCOCH_3$.

Lorsque $T_1$, $T_2$ ou $Z_3$ représentent un atome d'halogène, il s'agit de préférence d'un atome de fluor, de chlore ou de brome.

Lorsque $T_1$ ou $T_2$ représente un radical alkyle ou alkyloxy, il s'agit de préférence du radical méthyle, éthyle, propyle, méthoxy, éthoxy ou propoxy.

a, b, c et d représentent de préférence un atome de chlore ou de brome.

Lorsque D représente un atome d'halogène, il s'agit de préférence d'un atome de fluor, de chlore ou de brome.

Lorsque J représente un radical alkyle substitué par un ou plusieurs groupements fonctionnels, on entend de préférence par alkyle un radical renfermant de 1 à 8 atomes de carbone comme, par exemple, le radical méthyle, éthyle, propyle, isopropyle, butyle, isobutyle ou tert-butyle et par groupement fonctionnel l'un de ceux cités dans la demande européenne publiée sous le n° 50534.

J peut également représenter un radical alkyle substitué par un radical aryle, notamment radical phényle éventuellement substitué.

Lorsque J représente un radical alkyle substitué par un ou plusieurs groupements fonctionnels, on peut citer comme valeurs préférées de J, les radicaux :

$-(CH_2)_{n_1}-C(Hal)_3$ dans lequel $n_1$ est un entier de 1 à 8 et Hal un atome d'halogène, par exemple le radical $-CH_2-CCl_3$, $-CH_2-CF_3$, $-CH_2-CH_2-CCl_3$ ou $CH_2-CH_2-CF_3$ ;

$-(CH_2)_{n_2}-CH(Hal)_2$ dans lequel Hal est défini comme ci-dessus et $n_2$ est un nombre de 0 à 8, par exemple le radical $-CH_2-CHCl_2$, $-CH_2-CHF_2$ ou $-CHF_2$ ;

$-(CH_2)_{n_1}-CH_2(Hal)$ dans lequel $n_1$ et Hal sont définis comme ci-dessus par exemple le radical $-CH_2-CH_2Cl$ ou $-CH_2-CH_2F$ ;

$-C(CHal_3)_3$ dans lequel Hal est défini comme ci-dessus, par exemple le radical $-C(CF_3)_3$ le radical $-C(CF_3)_2-CCl_3$, $-C(CF_3)_2-CH_3$, $-C(CH_3)_2-CF_3$ ou $-C(CH_3)(CF_3)-CH_2-CH_3$, $-CH(CF_3)-CH_3$ ou $-CH(CF_3)_2$, $-C(CH_3)_2-CN$, $-CH(CH_3)-CN$ ou $-(CH_2)_n-CN$ dans lequel n est défini comme précédemment, $-CH(CN)-C(Hal)_3$ dans lequel Hal est défini comme précédemment,

par exemple le radical : $-CH(CN)-CCl_3$ ;

$-(CH_2)_{n_1}-OR_a$, dans lequel $n_1$ est défini comme précédemment et $R_a$ représente un atome d'hydrogène ou un radical alkyle linéaire ou ramifié, comportant de 1 à 8 atomes de carbone, par exemple le radical $-CH_2-OCH_3$, $-CH_2-CH_2-O-CH_3$, $-CH_2-CH_2-O-CH_2-CH_3$ ou $-CH_2-CH_2-OH$ ;

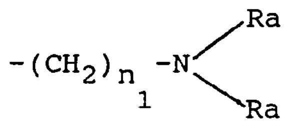

dans lequel $n_1$ et $R_a$ sont définis comme précédemment et les deux radicaux $R_a$ peuvent être différents entre eux, par exemple le radical :

$-CH_2-CH_2-NH-CH_3$, $-CH_2-CH_2-N(CH_3)_2$ ou

$-CH_2-CH_2-N(CH_3)-CH_2-CH_3$ ;

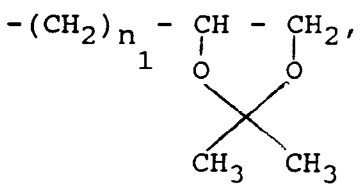

dans lequel $n_1$ est défini comme précédemment par exemple le radical :

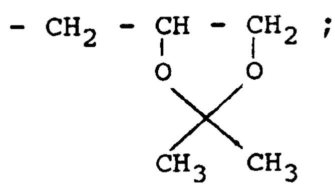

$-(CH_2)_{n_1}-CH(OH)-CH_2-OH$ dans lequel $n_1$ est défini comme précédemment par exemple le radical $-CH_2-CH(OH)-CH_2-OH$ ;

$-(CH_2)_{n_1}-O-THP$ dans lequel $n_1$ est défini comme précédemment et THP représente le radical 2-tétrahydropyrannyle, par exemple le radical : $-CH_2-O-THP$ ou $-CH_2-CH_2-O-THP$ ;

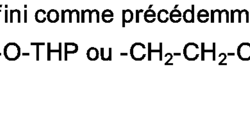

dans lequel $n_1$ est défini comme précédemment, par exemple le radical benzyle ou phénéthyle ;

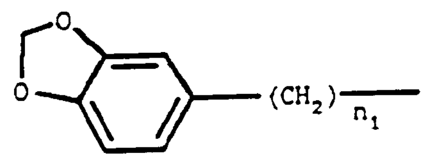

dans lequel $n_1$ est défini comme précédemment par exemple le radical :

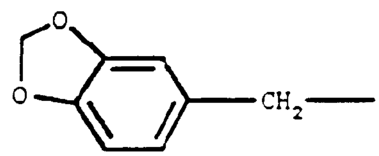

Lorsque J représente un radical aryle éventuellement substitué, il s'agit de préférence du radical phényle éventuellement substitué.

Lorsque J représente un radical hétérocyclique, il s'agit de préférence un radical pyridyle, furyle, thiényle, oxazolyle ou thiazolyle.

L'invention a particulièrement pour objet les composés de formule (I) dans lesquels Z représente un atome d'oxygène.

L'invention a plus particulièrement pour objet les composés de formule (I) dans lesquels l'un des radicaux $Y_1$ et $Y_2$ représentent un atome d'hydrogène.

L'invention a tout spécialement pour objet les composés de formule (I) dans lesquels celui des radicaux $Y_1$ ou $Y_2$ qui ne représente pas un atome d'hydrogène est en position 4 ou 5 ainsi que ceux dans lesquels le radical

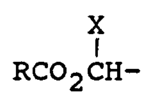

est en position 2.

Parmi les composés préférés de l'invention on peut citer les composés de formule (I) dans lesquels $Y_1$ ou $Y_2$ représente un radical alkyle renfermant jusqu'à 4 atomes de carbone éventuellement substitué par un ou plusieurs atomes de fluor, et notamment le radical $CHF_2$, ou encore le radical $CF_3$.

Parmi les composés préférés de l'invention, on peut également citer les composés de formule (I) dans lesquels l'un des radicaux $Y_1$ et $Y_2$ représente un radical $NO_2$, $-C{\equiv}CH$ ou $-CH_2-C{\equiv}CH$.

Parmi les composés préférés de l'invention, on peut également citer les composés de formule (I) dans lesquels X représente un atome d'hydrogène ou encore un radical éthynyle.

L'invention a plus particulièrement pour objet les composés de formule (I) dans lesquels R représente le reste d'un acide dérivé de l'acide cyclopropane carboxylique dans lequel la copule cyclopropanique est de structure 1 R cis.

Parmi les composés préférés de l'invention, on peut citer les composés de formule (I) dans lesquels R représente le radical :

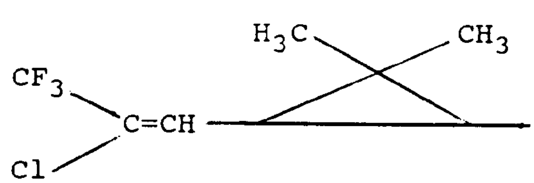

sous toutes ses formes stéréoisomères possibles, ainsi que leurs mélanges.

L'invention a comme composé préféré celui des exemples 1, 5, 7, 9, 10, 12, 18, 19, 26, 27, 28, 30 et 37 et plus particulièrement celui de l'exemple 2.

L'invention a également pour objet un procédé de préparation caractérisé en ce que l'on soumet un acide de formule (II) :

$$RCO_2H \quad (II)$$

dans laquelle R est défini comme précédemment ou un dérivé fonctionnel de cet acide à l'action d'un alcool de formule (III) :

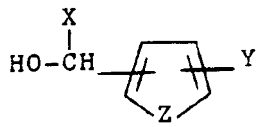

(III)

dans laquelle X, Y et Z sont définis comme précédemment ou d'un dérivé fonctionnel de cet alcool pour obtenir le composé de formule (I) correspondant.

Le dérivé fonctionnel d'acide utilisé est de préférence un chlorure d'acide.

Lorsque l'on fait réagir l'acide de formule (II) avec l'alcool de formule (III), on opère de préférence en présence de dicyclohexylcarbodiimide.

Les acides de formule (II) utilisés sont des produits connus utilisés dans la synthèse de composés pyréthrinoïdes.

Les alcools de formule (III) sont des produits nouveaux et sont en eux-mêmes un objet de la présente invention. Ils peuvent être préparés par analogie avec les produits dont la préparation est donnée ci-après en préparations 1 et 2, et qui peut être schématisé comme suit :

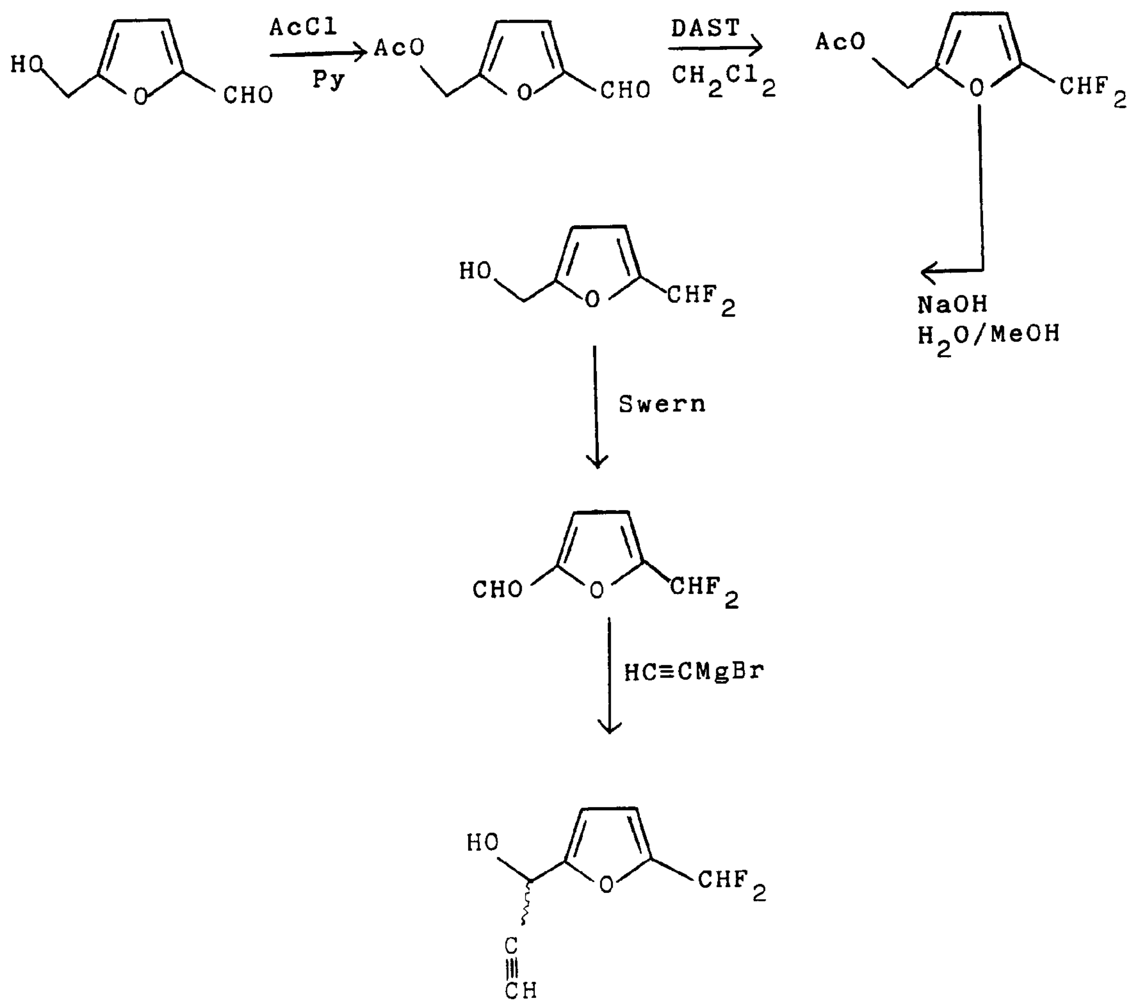


Les composés de formule (III) dans lesquels Z représente un atome de soufre, peuvent être préparés par

analogie avec le procédé suivant :

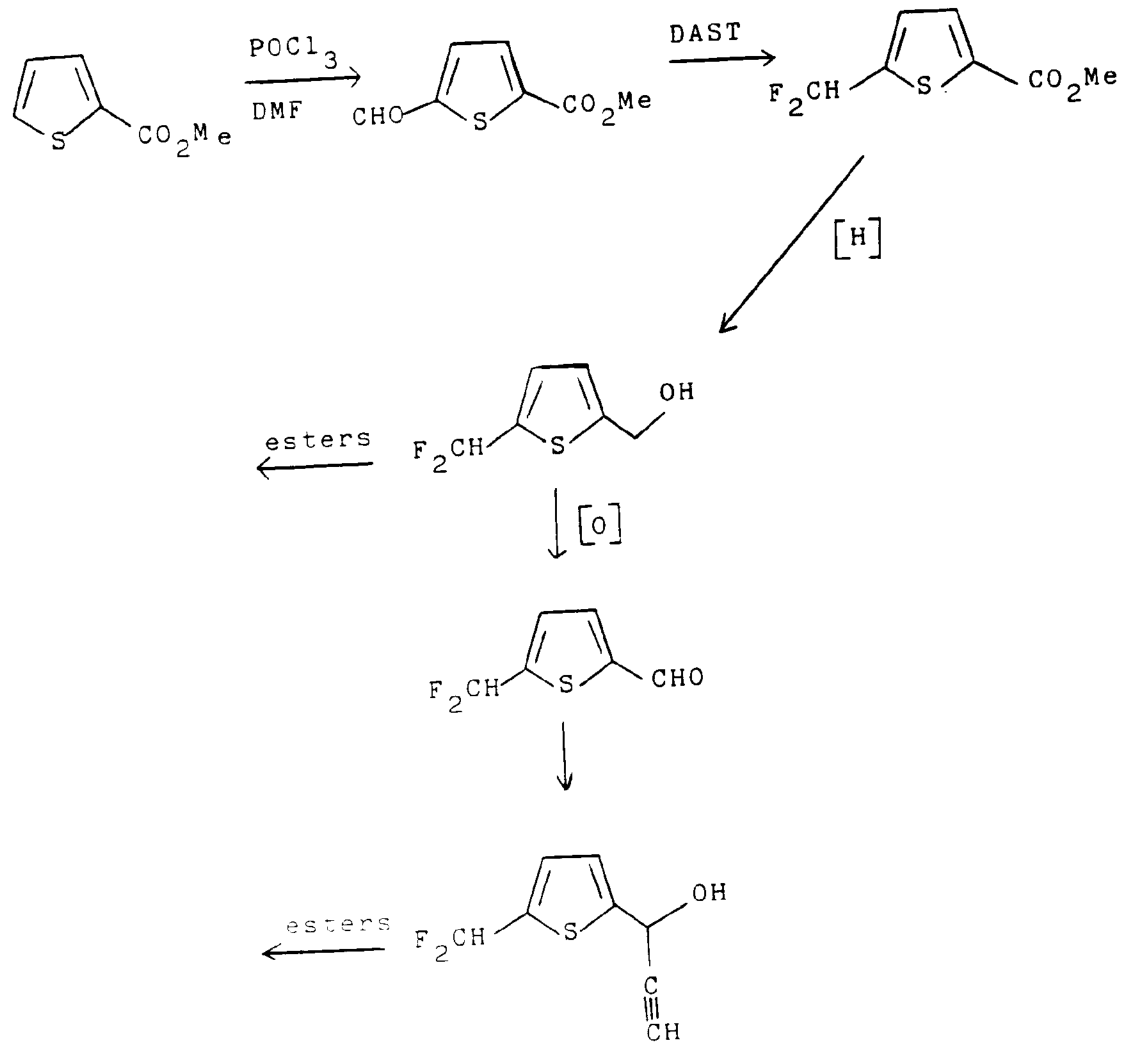


Les composés de formule (I) présentent d'intéressantes propriétés qui permettent leur utilisation dans la lutte contre les parasites. Il peut s'agir par exemple de la lutte contre les parasites des végétaux, les parasites des locaux et les parasites des animaux à sang chaud.

C'est ainsi que l'on peut utiliser les produits de l'invention pour lutter contre les insectes, les nématodes et les acariens parasites des végétaux et des animaux.

L'invention a notamment pour objet l'application des composés de formule (I) à la lutte contre les parasites des végétaux, les parasites des locaux et les parasites des animaux à sang chaud.

Les produits de formule (I) peuvent aussi être utilisés pour lutter contre les insectes et autres parasites du sol, par exemple les coléoptères, comme Diabrotica, les taupins et les vers blancs, les myriapodes comme les scutigérelles et les blaniules, et les diptères comme les cécydomies et les lépidoptères comme les noctuelles terricoles.

Ils sont utilisés à des doses comprises entre 10 g et 300 g de matière active à l'hectare.

Les produits de formule (I) peuvent également être utilisés pour lutter contre les insectes dans les locaux, pour lutter notamment contre les mouches, les moustiques et les blattes.

Les produits de formule (I) sont de plus photostables et sont peu toxiques pour les mammifères.

L'ensemble de ces propriétés fait que les produits de formule (I) correspondent parfaitement aux exigences de l'industrie agrochimique moderne : ils permettent de protéger les récoltes tout en préservant l'environnement.

Les produits de formule (I) peuvent aussi être utilisés pour lutter contre les acariens et les nématodes parasites des végétaux.

Les composés de formule (I) peuvent encore être utilisés pour lutter contre les acariens parasites des animaux, pour lutter par exemple contre les tiques et notamment les tiques de l'espèce de Boophilus, ceux de l'espèce Hyalomnia, ceux de l'espèce Amblyomnia et ceux de l'espèce Rhipicephalus ou pour lutter contre toutes sortes de gales et notamment la gale sarcoptique, la gale psoroptique et la gale chorioptique.

L'invention a donc également pour objet les compositions destinées à la lutte contre les parasites des animaux à sang chaud, les parasites des locaux et des végétaux, caractérisées en ce qu'elles renferment au moins l'un des produits de formule (I) définis ci-dessus et notamment les produits de formule (I) des exemples 1, 5, 7, 9, 10, 12, 18, 19, 26, 27, 28, 30 et 37 et plus particulièrement le produit de l'exemple 2.

L'invention a notamment pour objet les compositions insecticides renfermant comme principe actif au moins l'un des produits définis ci-dessus.

Ces compositions sont préparées selon les procédés usuels de l'industrie agrochimique ou de l'industrie vétérinaire ou de l'industrie des produits destinés à la nutrition animale.

Dans ces compositions destinées à l'usage agricole et à l'usage dans les locaux, la ou les matières actives peuvent être additionnées éventuellement d'un ou plusieurs autres agents pesticides. Ces compositions peuvent se présenter sous forme de poudres, granulés, suspensions, émulsions, solutions, solutions pour aérosols, bandes combustibles, appâts ou autres préparations employées classiquement pour l'utilisation de ce genre de composés.

Outre le principe actif, ces compositions contiennent, en général, un véhicule et/ou un agent tensio-actif, non ionique, assurant, en outre, une dispersion uniforme des substances constitutives du mélange. Le véhicule utilisé peut être un liquide, tel que l'eau, l'alcool, les hydrocarbures ou autres solvants organiques, une huile minérale, animale ou végétale, une poudre telle que le talc, les argiles, les silicates, le kieselguhr ou un solide combustible.

Les compositions insecticides selon l'invention contiennent de préférence de 0,005 % à 10 % en poids de matière active.

Selon un mode opératoire avantageux, pour un usage dans les locaux, les compositions selon l'invention sont utilisées sous forme de compositions fumigantes.

Les compositions selon l'invention peuvent alors être avantageusement constituées, pour la partie non active, d'un serpentin insecticide (ou coil) combustible, ou encore d'un substrat fibreux incombustible. Dans ce dernier cas, le fumigant obtenu après incorporation de la matière active est placé sur un appareil chauffant tel qu'un émanateur électrique.

Dans le cas où l'on utilise un serpentin insecticide, le support inerte peut être, par exemple, composé de marc de pyrèthre, poudre de Tabu (ou poudre de feuilles Machilus thumbergii), poudre de tige de pyrèthre, poudre de feuille de cèdre, poudre de bois (telle que de la sciure de pin) amidon et poudre de coque de noix de coco.

La dose de matière active peut alors être, par exemple, de 0,03 à 1 % en poids.

Dans le cas où l'on utilise un support fibreux incombustible, la dose de matière active peut alors être, par exemple, de 0,03 à 95 % en poids.

Les compositions selon l'invention pour un usage dans les locaux peuvent aussi être obtenues en préparant une huile pulvérisable à base de principe actif, cette huile imbibant la mèche d'une lampe et étant alors soumise à la combustion.

La concentration du principe actif incorporé à l'huile est, de préférence, de 0,03 à 95 % en poids.

L'invention a également pour objet les compositions acaricides et nématicides renfermant comme principe actif au moins un des produits de formule (I) définie ci-dessus.

Les compositions insecticides selon l'invention, comme les compositions acaricides et nématicides, peuvent être additionnées éventuellement d'un ou plusieurs autres agents pesticides. Les compositions acaricides et nématicides peuvent se présenter notamment sous forme de poudre, granulés, suspensions, émulsions, solutions.

Pour l'usage acaricide, on utilise de préférence des poudres mouillables, pour pulvérisation foliaire, contenant de 1 à 80 % en poids de principe actif ou des liquides pour pulvérisation foliaire contenant de 1 à 500 g/l de principe actif. On peut également employer des poudres pour poudrages foliaires contenant de 0,05 à 3 % de matière active.

Pour l'usage nématicide, on utilise de préférence des liquides pour traitement des sols contenant de 300 à 500 g/l de principe actif.

Les composés acaricides et nématicides selon l'invention sont utilisés, de préférence, à des doses comprises entre 1 et 100 g de matière active à l'hectare.

Pour exalter l'activité biologique des produits de l'invention on peut les additionner à des synergistes classiques utilisés en pareil cas tel que le 1-(2,5,8-trioxadodécyl) 2-propyl 4,5-méthylènedioxy benzène (ou butoxyde de pipéronyle) ou la N-(2-éthyl heptyl) bicyclo [2,2-1] 5-heptène-2,3-dicarboximide, ou le pipéronyl-bis-2-(2'-n-butoxy éthoxy) éthylacétal (ou tropital).

Les composés de formule (I) présentent une excellente tolérance générale, et l'invention a donc également pour objet les produits de formule (I), pour lutter notamment contre les affections créées par les tiques et les gales chez l'homme et l'animal.

Les produits de l'invention sont notamment utilisés pour lutter contre les poux à titre préventif ou curatif et pour lutter contre la gale.

Les produits de l'invention peuvent être administrés par voie externe, par vaporisation, par shampooing, par bain ou badigeonnage.

Les produits de l'invention à usage vétérinaire peuvent être également administrés par badigeonnage de l'épine dorsale selon la méthode dite méthode "pour-on".

On peut indiquer également que les produits de l'invention peuvent être utilisés comme biocides ou comme régulateurs de croissance.

L'invention a également pour objet les associations douées d'activité insecticide, acaricide ou nématicide, caractérisées en ce qu'elles contiennent comme matière active, d'une part un au moins des composés de formule générale (I), et d'autre part, un au moins des esters pyréthrinoïdes choisis dans le groupe constitué par les esters d'alléthrolone, d'alcool 3,4,5,6-tétrahydrophtalimido méthylique, d'alcool 5-benzyl 3-furyl méthylique, d'alcool 3-phénoxy benzylique et d'alcool alpha-cyano 3-phénoxy benzylique des acides chrysanthémiques, par les esters d'alcool 5-benzyl 3-furyl méthylique des acides 2,2-diméthyl 3-(2-oxo 3-tétrahydrothiophénylidène méthyl) cyclopropanecarboxyliques, par les esters d'alcool 3-phénoxy benzylique et d'alcool alpha-cyano 3-phénoxy benzylique des acides 2,2-diméthyl 3-(2,2-dichlorovinyl) cyclopropanecarboxyliques, par les esters d'alcool alpha-cyano 3-phénoxy benzylique d'acides 2,2-diméthyl 3-(2,2-dibromovinyl) cyclopropanecarboxyliques, par les esters d'alcool 3-phénoxy benzylique des acides 2-parachlorophényl 2-isopropyl acétiques, par les esters d'alléthrolone, d'alcool 3,4,5,6-tétrahydrophtalimidométhylique, d'alcool 5-benzyl 3-furyl méthylique, d'alcool 3-phénoxy benzylique et d'alcool alpha-cyano 3-phénoxy benzylique des acides 2,2-diméthyl 3-(1,2,2,2-tétrahaloéthyl) cyclopropanecarboxyliques, dans lesquels "halo" représente un atome de fluor, de chlore ou de brome, étant entendu que les composés (I) peuvent exister sous toutes leurs formes stéréoisomères possibles de même que les copules acides et alcools des esters pyréthrinoïdes ci-dessus.

Les exemples suivants illustrent l'invention sans toutefois la limiter.

**<u>EXEMPLE 1</u>: [1R[1alpha,3alpha (Z)]] 2,2-diméthyl-3-(3,3,3-trifluoro 2-chloro propényl) cyclopropane carboxylate de [5-(difluorométhyl)-2-furyl] méthyle.**

On refroidit à 5°C un mélange renfermant 800 mg de 5-difluorométhyl -2-furanméthanol, 25 $cm^3$ de chlorure de méthylène et 1,4 g d'acide 1R[1alpha,3alpha (Z)] 2,2-diméthyl 3-(3,3,3-trifluoro 2-chloro propényl) cyclopropane carboxylique. On ajoute à 5°C, une solution renfermant 1,2 g de DCC, 60 mg de DMAP, et 15 $cm^3$ de chlorure de méthylène. On laisse le mélange réactionnel revenir à la température ambiante, et maintient sous agitation pendant 2 heures. On ajoute 10 $cm^3$ d'hexane, agite pendant 5 minutes, filtre et concentre. On chromatographie le produit obtenu sur silice en éluant avec le mélange cyclohexane-acétate d'éthyle 95-5. On obtient 2,01 g d'un produit que l'on filtre et sèche. On obtient 1,42 g de produit recherché.

**<u>Préparation 1</u> : 5-(difluorométhyl)-2-furanméthanol.**

<u>Stade A</u> : 5-(acétyloxy) méthyl-2-furancarboxaldéhyde.

On ajoute à 5°C, 10,05 $cm^3$ de chlorure d'acétyle dans une solution renfermant 16,2 g de 5-(hydroxy méthyl) 2-furancarboxaldéhyde et 200 $cm^3$ de chlorure de méthylène. On ajoute ensuite 11,4 $cm^3$ de pyridine et 50 $cm^3$ de chlorure de méthylène. On maintient le mélange réactionnel sous agitation pendant 3 heures à 20°C. On traite avec une solution aqueuse de phosphate acide de sodium et extrait au chlorure de méthylène. On sèche et concentre. On obtient après chromatographie sur silice en éluant avec le mélange hexane-acétate d'éthyle (7-3), 19,35 g de produit recherché.

<u>Stade B</u> : Acétate de 5-(difluorométhyl)-2-furan méthanol.

On ajoute à 5°C une solution renfermant 7,26 $cm^3$ de DAST (diéthylamino sulfure trifluorure) et 30 $cm^3$ de chlorure de méthylène, dans une solution renfermant 10 g de produit préparé au stade A et 100 $cm^3$ de chlorure de méthylène. On agite le mélange réactionnel à 20°C pendant une demi heure et porte au reflux pendant 1 heure. On maintient sous agitation pendant 18 heures à 20°C et porte à reflux pendant 3 heures. On traite avec du carbonate acide de sodium et extrait au chlorure de méthylène. On sèche et concentre. On obtient 12 g d'un produit que l'on chromatographie sur silice en éluant avec le mélange hexane-acétate d'éthyle

8-2. On obtient ainsi 5,5 g du produit recherché.

Stade C : 5-(difluoro méthyl)-2-furanméthanol.

On ajoute 34,8 $cm^3$ d'une solution normale de soude dans une solution renfermant 5,5 g de produit préparé au stade précédent et 100 $cm^3$ de méthanol. On agite pendant 1 heure à 20°C. On traite avec une solution saturée de phosphate acide de sodium. On concentre et chromatographie sur silice le produit obtenu en éluant avec le mélange hexane-acétate d'éthyle 7-3. On obtient ainsi 3,6 g de produit recherché.

**EXEMPLE 2: [1R[1alpha,3alpha (Z)]] 2,2-diméthyl-3-(3,3,3-trifluoro 2-chloro propényl) cyclopropane carboxylate de [5-difluorométhyl 2-furyl] 2-propynyle.**

En opérant comme à l'exemple 1, à partir de 1,69 g l'acide 1R[1alpha,3alpha (Z)] 2,2-diméthyl 3-(3,3,3-trifluoro 2-chloro propényl) cyclopropane carboxylique et de 1,2 g [5-difluorométhyl 2-furyl] 2-propynol, on obtient après chromatographie 2,47 g du produit recherché.

**Préparation 2 : 5-(difluorométhyl) alpha-éthynyl 2-furanméthanol.**

Stade A : 5-difluorométhyl 2-furancarboxaldéhyde.

On ajoute à -60°C, sous atmosphère d'azote une solution renfermant 6,5 $cm^3$ de DMSO (diméthyl sulfoxyde) et 60 $cm^3$ de chlorure de méthylène dans une solution renfermant 3,84 $cm^3$ de chlorure d'oxalyle et 40 $cm^3$ de chlorure de méthylène. On maintient le mélange réactionnel sous agitation à -60°C et ajoute 3,6 g de 5-difluorométhyl 2-furanméthanol en solution dans 30 $cm^3$ de chlorure de méthylène. On agite pendant 2 heures à -60°C et ajoute en un quart d'heure une solution de 16,1 $cm^3$ de triéthylamine et 30 $cm^3$ de chlorure de méthylène. On maintient le mélange réactionnel sous agitation pendant une demi heure à -60°C, laisse la température remonter à -20°C et agite pendant une demi heure à cette température. On verse sur une solution de phosphate acide de sodium, extrait au chlorure de méthylène et sèche. On obtient 3,6 g de produit que l'on chromatographie sur silice en éluant avec le mélange hexane-acétate d'éthyle 8-2. On obtient après chromatographie 2,5 g de produit recherché.

Stade B : [5-difluorométhyl 2-furyl] 2-propynol.

On ajoute à 0°C en une heure 43 $cm^3$ d'une solution 0,5 M de bromure d'éthynyl magnésium dans le tétrahydrofuranne, dans une solution renfermant 2,5 g de produit préparé au stade A et 30 $cm^3$ de tétrahydrofuranne. On maintient le mélange réactionnel sous agitation à 0°C pendant 1 heure. On verse sur une solution aqueuse saturée en phosphate acide de sodium. On obtient 3 g de produit que l'on chromatographie sur silice en éluant avec le mélange hexane-acétate d'éthyle 7-3. On obtient 2,5 g de produit recherché.

En opérant comme dans l'exemple 1 ou 2 en utilisant l'alcool et l'acide appropriés, on a obtenu les produits suivants :

**EXEMPLE 3: [1R-[1alpha,3alpha(Z)]] 2,2-diméthyl 3-(2-fluoro 3-méthoxy 3-oxo 1-propényl) cyclopropane carboxylate de [5-(difluorométhyl) 2-furyl] méthyle.**

**EXEMPLE 4: [1R-[1alpha,3alpha(Z)]] 2,2-diméthyl 3-(2,2-difluoro cyclopropylidène) méthyl cyclopropane carboxylate de [5-(difluorométhyl) 2-furyl] méthyle.**

**EXEMPLE 5: [1R-[1alpha,3alpha(Z)]] 2,2-diméthyl 3-(2-fluoro 3-méthoxy 3-oxo 1-propényl) cyclopropane carboxylate de 1-[5-(difluorométhyl) 2-furyl] 2-propynyle.**

**EXEMPLE 6: [1R-[1alpha,3alpha(Z)]] 2,2-diméthyl 3-(2-fluoro 3-terbutoxy 3-oxo 1-propényl) cyclopropane carboxylate de 1-[5-(difluorométhyl) 2-furyl] méthyle.**

**EXEMPLE 7: [1R-[1alpha,3alpha(Z)]] 2,2-diméthyl 3-(2,2-dichloro éthényl) cyclopropane carboxylate de 1-[5-(difluorométhyl) 2-furyl] méthyle.**

**EXEMPLE 8: [1R-[1alpha,3bêta(Z)]] 2,2-diméthyl 3-[2-chloro 2-[(4-chlorophényl) éthényl] cyclopropane carboxylate de [5-(difluorométhyl) 2-furyl] méthyle.**

**EXEMPLE 9: [1R-[1alpha,3alpha(Z)]] 2,2-diméthyl 3-(2,2-difluoro cyclopropylidène) méthyl cyclopropane carboxylate de 1-[5-(difluorométhyl) 2-furyl] 2-propynyle.**

**EXEMPLE 10: [1R-[1alpha,3alpha(Z)]] 2,2-diméthyl 3-[3-(1-trifluorométhyl) (2,2,2-trifluoroéthoxy) 3-oxo 1-propényl] cyclopropane carboxylate de 1-[5-(difluorométhyl) 2-furyl] 2-propynyle.**

**EXEMPLE 11: [1S-[1alpha,3alpha]] 2-isopropyl 2-parachlorophényl acétate de 1-[5-(difluorométhyl) 2-furyl] 2-propynyle.**

**EXEMPLE 12: [1R-[1alpha,3alpha(Z)]] 2,2-diméthyl 3-[3,3,3-trifluoro 2-chloropropényl] cyclopropane carboxylate de (S) 1-[5-(difluorométhyl) 2-furyl] 2-propynyle et [1R-[1alpha, 3alpha(Z)]] 2,2-diméthyl 3-[3,3,3-trifluoro 2-chloropropényl] cyclopropane carboxylate de (R) 1-[5-(difluorométhyl) 2-furyl] 2-propynyle.**

**EXEMPLE 13: [1R-[1alpha,3bêta(Z)]] 2,2-diméthyl 3-(2-fluoro 1-propényl) cyclopropane carboxylate de 1-[5-(difluorométhyl) 2-furyl] 2-propynyle.**

**EXEMPLE 14: [1R-[1alpha,3alpha(Z)]] 2,2-diméthyl 3-[3,3,3-trifluoro 2-chloropropényl] cyclopropane carboxylate de 1-[4-(difluorométhyl) 2-furyl] 2-propynyle.**

**EXEMPLE 15: [1R-[1alpha,3alpha(Z)]] 2,2-diméthyl 3-[3,3,3-trifluoro 2-chloropropényl] cyclopropane carboxylate de [2-(trifluorométhyl) 3-furyl] méthyle.**

Rf = 0,25 (hexane-éther isopropylique 95-5).

**EXEMPLE 16: [1R-[1alpha,3alpha(Z)]] 2,2-diméthyl 3-(2-fluoro 3-méthoxy 3-oxo 1-propényl) cyclopropane carboxylate de [2-(trifluorométhyl) 3-furyl] méthyle.**

Rf = 0,15 (hexane-AcOEt 9-1).

**EXEMPLE 17: [1R-[1alpha,3alpha(Z)]] 2,2-diméthyl 3-[3,3,3-trifluoro 2-chloropropényl] cyclopropane carboxylate de [2-(trifluorométhyl) 5-propynyl 3-furyl] méthyle.**

Rf = 0,15 (hexane-AcOEt 95-5).

**EXEMPLE 18: [1R-[1alpha,3alpha(Z)]] 2,2-diméthyl 3-[3,3,3-trifluoro 2-chloropropényl] cyclopropane carboxylate de 1-[5-(trifluorométhyl) 2-furyl] 2-propynyle.**

Rf = 0,2 (hexane-AcOEt 95-5).

**EXEMPLE 19: [1R-[1alpha,3alpha(Z)]] 2,2-diméthyl 3-(2-fluoro 3-méthoxy 3-oxo 1-propényl) cyclopropane carboxylate de 1-[5-(trifluorométhyl) 2-furyl] 2-propynyle.**

Rf = 0,15 (hexane-AcOEt 9-1).

**EXEMPLE 20: [1R-[1alpha,3alpha(Z)]] 2,2-diméthyl 3-[3,3,3-trifluoro 2-chloropropényl] cyclopropane carboxylate de 1-(2-furyl) 2-propynyle.**

Rf = 0,25 (hexane-AcOEt 95-5).

**EXEMPLE 21: [1R-[1alpha,3alpha(Z)]] 2,2-diméthyl 3-(2-fluoro 3-méthoxy 3-oxo 1-propényl) cyclopropane carboxylate de 1-(2-furyl) 2-propynyle.**

Rf = 0,15 (hexane-AcOEt 9-1).

**EXEMPLE 22: [1R-[1alpha,3alpha(Z)]] 2,2-diméthyl 3-[3,3,3-trifluoro 2-chloropropényl] cyclopropane carboxylate de [5-(méthoxyméthyle) 2-furyl] méthyle.**

Rf = 0,3 (hexane-AcOEt 8-2).

**EXEMPLE 23: [1R-[1alpha,3alpha(Z)]] 2,2-diméthyl 3-(2-fluoro 3-méthoxy 3-oxo 1-propényl) cyclopropane carboxylate de (5-méthoxyméthyl 2-furyl) méthyle.**

Rf = 0,17 (hexane-AcOEt 8-2).

**EXEMPLE 24: [1R-[1alpha,3alpha(Z)]] 2,2-diméthyl 3-[3,3,3-trifluoro 2-chloropropényl] cyclopropane carboxylate de 1-[5-(méthoxyméthyle) 2-furyl] 2-propynyle.**

Rf = 0,24 (hexane-AcOEt 8-2).

**EXEMPLE 25: [1R-[1alpha,3alpha(Z)]] 2,2-diméthyl 3-(2-fluoro 3-méthoxy 3-oxo 1-propényl) cyclopropane carboxylate de 1-[5-((méthoxyméthyle) 2-furyl] 2-propynyle.**

Rf = 0,15 (hexane-AcOEt 8-2).

**EXEMPLE 26: [1R-[1alpha,3alpha(Z)]] 2,2-diméthyl 3-[3,3,3-trifluoro 2-chloropropényl] cyclopropane carboxylate de (5-éthynyl 2-furyl) méthyle.**

Rf = 0,23 (hexane-AcOEt 95-5).

**EXEMPLE 27: [1R-[1alpha,3alpha(Z)]] 2,2-diméthyl 3-[3,3,3-trifluoro 2-chloropropényl] cyclopropane carboxylate de 1-(5-éthynyl 2-furyl) 2-propynyle.**

Rf = 0,24 (hexane-AcOEt 8-2).

**EXEMPLE 28: [1R-[1alpha,3alpha(Z)]] 2,2-diméthyl 3-[3,3,3-trifluoro 2-chloropropényl] cyclopropane carboxylate de [2-(difluorométhyl) 4-furyl] méthyle.**

Rf = 0,2 (hexane-AcOEt 95-5).

**EXEMPLE 29: [1R-[1alpha,3alpha(Z)]] 2,2-diméthyl 3-(2-fluoro 3-méthoxy 3-oxo 1-propényl) cyclopropane carboxylate de [2-(difluorométhyl) 4-furyl] méthyle.**

Rf = 0,25 (hexane-AcOEt 8-2).

**EXEMPLE 30: [1R-[1alpha,3alpha(Z)]] 2,2-diméthyl 3-[3,3,3-trifluoro 2-chloropropényl] cyclopropane carboxylate de 1-[2-(difluorométhyl) 4-furyl] 2-propynyle.**

Rf = 0,15 (hexane-AcOEt 95-5).

**EXEMPLE 31: [1R-[1alpha,3alpha(Z)]] 2,2-diméthyl 3-(2-fluoro 3-méthoxy 3-oxo 1-propényl) cyclopropane carboxylate de 1-[2-(difluorométhyl) 4-furyl] 2-propynyle.**

Rf = 0,15 (hexane-éther isopropylique 8-2).

**EXEMPLE 32: [1R-[1alpha,3alpha(Z)]] 2,2-diméthyl 3-[3,3,3-trifluoro 2-chloropropényl] cyclopropane carboxylate de [2-(difluorométhyl) 3-furyl] méthyle.**

Rf = 0,25 (hexane-AcOEt 95-5).

**EXEMPLE 33: [1R-[1alpha,3alpha(Z)]] 2,2-diméthyl 3-[3,3,3-trifluoro 2-chloropropényl] cyclopropane carboxylate de 1-[5-nitro 2-furyl] 2-propynyle.**

Rf = 0,35 (hexane-AcOEt 7-3).

**EXEMPLE 34: [1R-[1alpha,3alpha(Z)]] 2,2-diméthyl 3-[3,3,3-trifluoro 2-chloropropényl] cyclopropane carboxylate de [5-propynyl 2-furyl] méthyle.**

**EXEMPLE 35: [1R-[1alpha,3alpha(Z)]] 2,2-diméthyl 3-(2-fluoro 3-méthoxy 3-oxo 1-propényl) cyclopropane carboxylate de [5-propynyl 2-furyl] méthyle.**

Rf = 0,12 (hexane-AcOEt 9-1).

**EXEMPLE 36: [1R-[1alpha,3alpha(Z)]] 2,2-diméthyl 3-(2-fluoro 3-méthoxy 3-oxo 1-propényl) cyclopropane carboxylate de [5-(hydroxyméthyl) 2-furyl] méthyle.**

Rf = 0,3 (hexane-AcOEt 5-5).

**EXEMPLE 37: [1R-[1alpha,3alpha(Z)]] 2,2-diméthyl 3-[3,3,3-trifluoro 2-chloropropényl] cyclopropane carboxylate de [5-(trifluorométhyl) 2-furyl] méthyle.**

**EXEMPLE 38: [1R-[1alpha,3alpha(Z)]] 2,2-diméthyl 3-[3,3,3-trifluoro 2-chloropropényl] cyclopropane carboxylate de (5-cyano 2-furyl) méthyle.**

Rf = 0,27 (hexane-AcOEt 8-2).

**EXEMPLE 39: [1R-[1alpha,3alpha(Z)]] 2,2-diméthyl 3-[3,3,3-trifluoro 2-chloropropényl] cyclopropane carboxylate de 1-(3-thiényl) 2-propynyle.**

Rf = 0,2 (hexane-AcOEt 95-5).

**EXEMPLE 40: [1R-[1alpha,3alpha(Z)]] 2,2-diméthyl 3-(2-fluoro 3-méthoxy 3-oxo 1-propényl) cyclopropane carboxylate de 1-(3-thiényl) 2-propynyle.**

Rf = 0,35 (hexane-AcOEt 8-2).

**EXEMPLE 41: [1R-[1alpha,3alpha(Z)]] 2,2-diméthyl 3-[3,3,3-trifluoro 2-chloropropényl] cyclopropane carboxylate de 1-(2-thiényl) 2-propynyle.**

Rf = 0,25 (hexane-AcOEt 95-5).

**EXEMPLE 42: [1R-[1alpha,3alpha(Z)]] 2,2-diméthyl 3-(2-fluoro 3-méthoxy 3-oxo 1-propényl) cyclopropane carboxylate de 1-(2-thiényl) 2-propynyle.**

Rf = 0,25 (hexane-AcOEt 8-2).

**EXEMPLE 43: [1R-[1alpha,3alpha(Z)]] 2,2-diméthyl 3-[3,3,3-trifluoro 2-chloropropényl] cyclopropane carboxylate de [5-(difluorométhyl) 2-thiényl] méthyle.**

Rf = 0,2 (hexane-éther isopropylique 95-5).

**EXEMPLE 44: [1R-[1alpha,3alpha(Z)]] 2,2-diméthyl 3-[3,3,3-trifluoro 2-chloropropényl] cyclopropane carboxylate de 1-[5-(difluorométhyl) 2-thiényl] 2- propynyle.**

Rf = 0,4 (hexane-AcOEt 8-2).

**EXEMPLE 45: [1R-[1alpha,3bêta(Z)]] 2,2-diméthyl 3-(2-fluoro 3-méthoxy 3-oxo 1-propényl) cyclopropane carboxylate de 1-[5-(difluorométhyl) 2-thiényl] 2-propynyle.**

Rf = 0,23 (hexane-AcOEt 8-2).

**EXEMPLE 46: [1R-[1alpha,3alpha(Z)]] 2,2-diméthyl 3-[3,3,3-trifluoro 2-chloropropényl] cyclopropane carboxylate de 1-[3-chloro 5-(difluorométhyl) 2-thiényl] 2-propynyle.**

Rf = 0,12 (hexane-AcOEt 95-5).

**Préparation 3: [4-trifluorométhyl 2-furyl] 2-propynol.**

Stade A : 2-méthyl 4-hydroxy 4-trifluorométhyl 4,5-dihydrofuranne 3-carboxylate d'éthyle.

On mélange 18,8 g d'hydrure de sodium dans 175 $cm^3$ d'éthanol puis introduit cette solution en 3 heures dans le mélange comprenant 51 g d'acétyl acétate d'éthyle, 74,8 g de 1-bromo 3,3,3-trifluoroacétone et 200 $cm^3$ de toluène en maintenant la température inférieure à +10°C. On agite 18 heures, ajoute une solution aqueuse de bicarbonate de sodium, agite, extrait à l'éther isopropylique, sèche, évapore le solvant et récupère 62 g de produit attendu (Eb = 64-72°C).

Stade B : 2-méthyl 4-trifluorométhyl 3-furanne carboxylate d'éthyle.

On chauffe 7 heures au reflux 10 g de produit obtenu au stade A dans 50 $cm^3$ de toluène en présence de 0,5 g d'acide paratoluène sulfonique. On laisse revenir à température ambiante, évapore le solvant, chromatographie sur silice (éluant : n-hexane-acétate d'éthyle 95-5) et récupère 6,95 g de produit attendu.

Stade C : Acide 2-méthyl 4-trifluorométhyl 3-furanne carboxylique.

On chauffe 2 heures et demie au reflux 18 g de produit obtenu au stade B dans 42 $cm^3$ d'eau et 126 $cm^3$ d'éthanol en présence de 3,55 g de soude. On laisse revenir à température ambiante, ajoute de l'acide chlorhydrique 2N jusqu'à neutralité, concentre partiellement, ajoute de la soude 2N, extrait à l'éther isopropylique, évapore le solvant et obtient 14,25 g de produit attendu. F = 140°C.

Stade D : 2-méthyl 4-trifluorométhylfuranne.

On chauffe à 240°C, 13,45 g pendant 30 minutes de produit obtenu au stade C en présence de 0,6 g de sulfate de cuivre II et 10 $cm^3$ de quinoléine. On sèche le milieu réactionnel, distille sous pression atmosphérique et obtient 6,2 g de produit attendu. Eb : 72-74°C.

Stade E : 2-bromométhyl 4-trifluorométhylfuranne.

On chauffe 1 heure et demie au reflux 6 g du produit du stade D dans 50 $cm^3$ de tétrachlorure de carbone, en présence de 7,2 g de N-bromosuccinimide et 0,3 g d'azobisisobutyronitrile, on laisse revenir à température

ambiante, filtre, évapore le solvant, chromatographie le résidu sur silice (éluant : éther-éther isopropylique 98-2) et obtient 5,5 g de produit attendu.

Stade F : 4-trifluorométhyl 2-furancarboxaldéhyde.

On chauffe 1 heure au reflux 2,95 g de produit obtenu au stade E, 9 $cm^3$ d'eau et 3,6 g d'hexaméthylène tétramine. On ajoute 9 $cm^3$ d'acide chlorhydrique concentré, maintient le reflux pendant 1 heure supplémentaire, laisse revenir à température ambiante, dilue à l'eau, extrait au chlorure de méthylène, lave à l'eau, sèche, évapore le solvant et obtient 1,9 g de produit attendu.

Stade G : 1-[4-(trifluorométhyl) 2-furyl] 2-propynol.

On refroidit à 0°/+5°C, 1,8 g de produit obtenu au stade F dans 20 $cm^3$ de tétrahydrofuranne et ajoute en 30 minutes 24 $cm^3$ de bromure d'éthynyl magnésium. On laisse revenir à température ambiante, verse dans une solution aqueuse saturée en bicarbonate de sodium, extrait au chlorure de méthylène, sèche, évapore le solvant et obtient 0,8 g de produit attendu après chromatographie sur silice (éluant : chlorure de méthylène).

**Préparation 4: 2-trifluorométhyl 3-furan méthanol.**

Stade A : 2-hydroxy 2-trifluorométhyl 2,5-dihydrofuranne 3-carboxylate d'éthyle et 4-hydroxy 2-trifluorométhyl 4,5-dihydrofuranne 3-carboxylate d'éthyle (mélange).

On ajoute en 30 minutes à 20°C, 210 $cm^3$ de chloroacétaldéhyde à 36,5 $cm^3$ de 4,4,4-trifluoroacétoacétate d'éthyle dans 600 $cm^3$ de pyridine et maintient 52 heures sous agitation. On verse le milieu réactionnel dans 650 $cm^3$ d'acide chlorhydrique concentré et glace, agite 5 minutes, extrait à l'éther isopropylique, sèche et évapore le solvant. Après chromatographie sur silice (éluant : hexane-acétate d'éthyle 7-3), on obtient 43 g de produit attendu.

Stade B : 2-trifluorométhyl 3-furan carboxylate d'éthyle.

On chauffe 2 heures et demie au reflux 43 g du produit obtenu au stade A dans 250 $cm^3$ de toluène et 3,1 g d'acide paratoluène sulfonique ; on laisse refroidir, chromatographie le milieu réactionnel sur silice (éluant : hexane-acétate d'éthyle 9-1) et après évaporation du solvant à 40°C sous 80 mm de Hg, on obtient 20 g de produit attendu.

Stade C : 2-trifluorométhyl 3-furan méthanol.

On refroidit à -60°C 20 g du produit obtenu au stade B dans 200 $cm^3$ de toluène, ajoute goutte à goutte 160 $cm^3$ d'hydrure de diisobutylaluminium, agite 1 heure et demie à -60°C ; on verse sur du tartrate double de sodium et potassium, extrait au chlorure de méthylène, évapore le solvant, chromatographie le résidu sur silice (éluant : hexane-acétate d'éthyle 7-3) et obtient 16 g de produit attendu.

**Préparation 5: 2-trifluorométhyl 5-(2-propynyl) 3-furanne méthanol.**

Stade A : 4-oxo 2-(2,2,2-trifluoro 1-oxoéthyl) pentanoate d'éthyle.

On mélange 36,8 g de 4,4,4-trifluoroacétoacétate d'éthyle dans 400 $cm^3$ de tétrahydrofuranne, ajoute à 15°C en 30 minutes 9,6 g d'hydrure de sodium, maintient sous agitation à 20°C pendant 1 heure, ajoute 0,4 g d'iodure de potassium et 100 $cm^3$ d'acétone, ajoute goutte à goutte en 15 minutes 21 g de chloroacétone dans 75 $cm^3$ de tétrahydrofuranne, chauffe 96 heures au reflux. On distille et obtient 24,4 g de produit attendu. Eb : 65°C (0,2 mbar).

Stade B : 2-trifluorométhyl 5-méthyl 3-furanne carboxylate d'éthyle.

On mélange 24 g de produit obtenu au stade A dans 150 $cm^3$ de toluène, ajoute 1,85 g d'acide paratoluène sulfonique et chauffe 20 heures au reflux. On évapore le solvant sous pression réduite à 40°C, chromatographie sur silice (éluant : hexane-acétate d'éthyle 95-5) et obtient 13,5 g de produit attendu.

Stade C : 2-trifluorométhyl 5-bromométhyl 3-furanne carboxylate d'éthyle.

On opère comme dans la préparation 3 stade E à partir de 12,4 g de produit obtenu au stade B et obtient 12,8 g de produit attendu.

Stade D : 2-trifluorométhyl 5-bromométhyl 3-furanne méthanol.

On opère comme à la préparation 4 stade C à partir de 4,65 g de produit obtenu au stade C et obtient 4 g d'alcool attendu.

Stade E : 2-trifluorométhyl 5-bromométhyl 3-tétrahydropyranyloxy méthyle furanne.

On ajoute 2,82 $cm^3$ de dihydropyrane et 250 mg d'acide paratoluène sulfonique à 4 g de produit obtenu au stade D dans 80 $cm^3$ d'éther. On agite 3 heures à 20°C, évapore le solvant sous pression réduite, chromatographie le résidu sur silice (éluant : hexane-acétate d'éthyle 9-1) et obtient 4,8 g de produit attendu.

Stade F : 2-trifluorométhyl 5-(2-propynyl) 3-tétrahydropyranyloxy méthyle furanne.

A 6,18 g de produit préparé comme au stade E dans 15 $cm^3$ de tétrahydrofuranne, on ajoute 90 $cm^3$ de bromure d'éthynyl magnésium en solution 1M dans le tétrahydrofuranne et 1,8 g de chlorure de cuivre. On chauffe 18 heures au reflux, refroidit à 20°C, verse dans une solution aqueuse saturée en phosphate acide de sodium, extrait à l'éther isopropylique, sèche et évapore le solvant, chromatographie le résidu sur silice (éluant : hexane-éther isopropylique 9-1) et obtient 1,5 g de produit attendu.

Stade G : 2-trifluorométhyl 5-(2-propynyl) 3-furanne méthanol.

On mélange 1,1 g de produit obtenu au stade F dans 25 $cm^3$ de méthanol, ajoute 50 mg d'acide paratoluène sulfonique, agite 2 heures à 20°C. On verse dans l'eau glacée, extrait à l'éther isopropylique, sèche et évapore le solvant. On chromatographie le résidu sur silice (éluant : hexane-acétate d'éthyle 7-3) et obtient 600 mg d'alcool attendu.

**Préparation 6: 1-(5-trifluorométhyl 2-furyl) 2-propynol.**

Stade A : Acide 2-trifluorométhyl 5-méthyl 3-furan carboxylique.

On chauffe 1 heure au reflux 13 g de produit obtenu comme au stade B de la préparation 5, 100 $cm^3$ de méthanol et 38,5 $cm^3$ de soude 2N ; on évapore le solvant, reprend dans l'eau, lave avec 50 $cm^3$ d'éther isopropylique, acidifie au moyen de 6,5 $cm^3$ d'acide chlorhydrique et essore les cristaux formés, les lave à l'eau, sèche à 20°C sous pression réduite et obtient 10,33 g de produit attendu.

Stade B : 5-trifluorométhyl 2-méthyl furanne.

On opère comme au stade D de la préparation 3 à partir de 5,3 g d'acide obtenu au stade A. On obtient 3,41 g de produit attendu.

Stade C : 5-trifluorométhyl 2-bromométhyl furanne.

On opère comme au stade E de la préparation 3 à partir de 3, 41 g du produit obtenu au stade B. On obtient après chromatographie sur silice (éluant : hexane-éther isopropylique 95-5) 3,5 g de produit attendu.

Stade D : 5-trifluorométhyl 2-furanne carboxaldéhyde.

A 2,5 g de produit obtenu au stade D dans 25 $cm^3$ de chlorure de méthylène, on ajoute en 15 minutes 5,9 g de N-oxyde N-méthyl morpholine dans 25 $cm^3$ de chlorure de méthylène et 12,5 $cm^3$ de diméthyl sulfoxyde. On agite 5 heures à 20°C, verse dans une solution aqueuse de phosphate acide de sodium, extrait au chlorure de méthylène, lave à l'eau, sèche et évapore le solvant à 30°C (620 mbar). Après chromatographie sur silice (éluant : chlorure de méthylène), on obtient le produit brut utilisé tel quel au stade suivant.

Stade E : 1-(5-trifluorométhyl 2-furyl) 2-propynol.

On refroidit à +5°C le mélange comprenant 1,78 g environ du produit obtenu au stade D et 20 $cm^3$ environ de chlorure de méthylène, ajoute en 15 minutes 27 $cm^3$ de bromure d'éthynyl magnésium en solution 0,5M dans le tétrahydrofuranne. On agite 15 minutes à +5°C, verse dans une solution aqueuse de phosphate acide de sodium, extrait au chlorure de méthylène, sèche, évapore le solvant à 30°C, chromatographie le résidu sur silice (éluant : chlorure de méthylène) et récupère 0,87 g de produit attendu.

**Préparation 7: 1-(2-furyl) 2-propynol.**

On mélange 2 g de 2-furaldéhyde dans 30 $cm^3$ de tétrahydrofuranne, refroidit à 0°C, ajoute goutte à goutte en 15 minutes 50 $cm^3$ d'une solution 0,5M de bromure d'éthynyl magnésium dans le tétrahydrofuranne, agite 1 heure à 0°C, verse dans une solution aqueuse saturée en phosphate acide de sodium, extrait à l'éther isopropylique, sèche et évapore le solvant. On obtient 2,5 g de produit attendu après chromatographie sur silice (éluant : hexane-acétate d'éthyle 75-25).

**Préparation 8 : (5-méthoxyméthyl 2-furanne) méthanol.**

On dissout à 20°C, 6,4 g de 2,5-furandiméthanol dans 120 $cm^3$ de tétrahydrofuranne et ajoute en 30 minutes 2,4 g d'hydrure de sodium. On agite 2 heures, ajoute 60 $cm^3$ de diméthylformamide et 4,7 $cm^3$ de sulfate de diméthyle, agite 16 heures à 20°C, verse dans l'eau glacée, acidifie par de l'acide chlorhydrique. On extrait à l'éther isopropylique, lave à l'eau, sèche et évapore le solvant, chromatographie sur silice (éluant : hexane-acétate d'éthyle 5-5) et obtient 2,97 g de produit attendu.

**Préparation 9: 1-(5-méthoxyméthyl 2-furyl) 2-propynol.**

Stade A : 5-méthoxyméthyl 2-furan carboxaldéhyde.

A une suspension de 5,45 g de chlorochromate de pyridinium dans 50 $cm^3$ de chlorure de méthylène, on ajoute en 15 minutes 3 g de produit obtenu comme à la préparation 8 dans 30 $cm^3$ de chlorure de méthylène, agite 2 heures à 20°C, filtre, évapore le solvant, reprend le résidu dans 10 $cm^3$ de chlorure de méthylène, chromatographie sur silice (éluant : acétate d'éthyle-hexane 5-5) et obtient 1 g de produit attendu.

Stade B : 1-(5-méthoxyméthyl 2-furyl) 2-propynol.

On opère comme au stade B de la préparation 2 à partir de 1 g du produit obtenu au stade A et obtient 1,16 g de produit attendu.

**Préparation 10: 5-éthynyl 2-furanne méthanol.**

Stade A : 5-bromo 2-furan carboxylate d'éthyle.

On chauffe 24 heures au reflux 10 g d'acide 5-bromo 2-furanne carboxylique, dans 200 $cm^3$ d'éthanol en présence de 2 $cm^3$ de chlorure de thionyle. On évapore le solvant, verse dans une solution aqueuse de bicarbonate de sodium, extrait à l'acétate d'éthyle, sèche, évapore le solvant et obtient 8,5 g de produit attendu.

Stade B : 5-(triméthylsilyléthynyl) 2-furanne carboxylate d'éthyle.

On mélange 5 g de produit obtenu au stade A, 15 $cm^3$ d'acétonitrile, 15 $cm^3$ de triéthylamine, 4,5 $cm^3$ d'éthynyl triméthylsilane, 170 mg de chlorure de triphénylephosphine palladium et 30 mg d'iodure de cuivre. On chauffe à 40°C la solution obtenue pendant 2 heures et demie, laisse revenir à température ambiante et verse sur une solution aqueuse de phosphate acide de sodium. On extrait à l'acétate d'éthyle, lave à l'eau, sèche et évapore le solvant. Après chromatographie sur silice (éluant : hexane-acétate d'éthyle 95-5), on obtient 4,75 g de produit attendu.

Stade C : 5-éthynyl 2-furanne méthanol.

On opère comme indiqué à la préparation 4 stade C à partir de 4 g de produit obtenu au stade B ci-dessus.

On obtient le produit attendu.

**Préparation 11: 1-(5-éthynyl 2-furyl) 2-propynol.**

Stade A : 5-éthynyl 2-furanne carboxaldéhyde.

On opère comme au stade A de la préparation 2 à partir de 1,7 g de l'alcool obtenu à la préparation 10 et recueille 1,2 g de produit attendu.

Stade B : 1-(5-éthynyl 2-furyl) 2-propynol.

En opérant comme au stade B de la préparation 2 à partir de 1,2 g de l'aldéhyde préparé au stade A, on obtient 1,05 g de produit attendu.

**Préparation 12: 5-difluorométhyl 3-furanne méthanol.**

Stade A : 5-formyl 3-furanne carboxylate d'éthyle et 2-formyl 3-furanne carboxylate d'éthyle.

On ajoute à +5°C 9 $cm^3$ d'oxychlorure de phosphore dans 10 $cm^3$ de diméthylformamide, agite 40 minutes, laisse revenir à température ambiante 10 g de 3-furanne carboxylate d'éthyle puis chauffe 3 heures et demie à 100°-105°C, verse dans 300 $cm^3$ d'une solution de carbonate de sodium et 300 $cm^3$ de chlorure de méthylène, évapore le solvant, chromatographie sur silice (éluant : hexane-acétate d'éthyle 8-2) et obtient 6,93 g de produit attendu.

Stade B : 5-difluorométhyl furanne 3-carboxylate d'éthyle.

On opère comme au stade B de la préparation 1 à partir de 10,2 g d'aldéhydes préparés comme au stade A et 10 $cm^3$ de DAST. On obtient 7,9 g de produit attendu après chromatographie sur silice (éluant : hexane-acétate d'éthyle 9-1) et 1,2 g de l'ester 2-difluorométhylique correspondant.

Stade C : 5-difluorométhyl 3-furanne méthanol.

On opère comme au stade C de la préparation 4 à partir de 7,9 g de produit obtenu au stade B et obtient 4,8 g de produit attendu.

**Préparation 13: 1-(5-difluorométhyl 3-furyl) 2-propynol.**

Stade A : 5-difluorométhyl 3-furanne carboxaldéhyde.

On ajoute 4,8 g d'alcool obtenu à la préparation 12 dans 55 $cm^3$ de chlorure de méthylène à 8,4 g de chlorochromate de pyridinium dans 90 $cm^3$ de chlorure de méthylène. On agite 90 minutes, filtre, évapore le solvant, chromatographie le résidu sur silice (éluant : hexane-acétate d'éthyle 7-3) et obtient 3,2 g d'aldéhyde attendu.

Stade B : 1-(5-difluorométhyl 3-furyl) 2-propynol.

On opère comme au stade B de la préparation 2 à partir de 3,2 g de l'aldéhyde obtenu au stade A. On obtient 3,8 g de produit attendu.

**Préparation 14: 2-difluorométhyl 3-furanne méthanol.**

On opère comme au stade C de la préparation 4 à partir de 1,9 g de l'ester approprié obtenu à la préparation 12 stade B. On obtient 1 g d'alcool attendu.

**Préparation 15: 1-(5-nitro 2-furyl) 2-propynol.**

On opère comme au stade B de la préparation 2 à partir de 2,82 g de 5-nitro 2-furaldéhyde et 45 $cm^3$ de bromure d'éthynyl magnésium. On obtient 1,84 g de produit attendu après chromatographie sur silice (éluant :

hexane-acétate d'éthyle 8-2).

**Préparation 16: 5-hydroxyméthyl 2-furanne carbonitrile.**

Stade A : 5-formyl 2-furanne carbonitrile.

On refroidit à -60°C, 75 $cm^3$ de tétrahydrofuranne et 22,6 $cm^3$ de diisopropylamine et ajoute en 25 minutes 100 $cm^3$ de n-butyllithium à 1,6M dans l'hexane. On agite pendant 1 heure, ajoute 13,02 g de 2-furanne carbonitrile dans 50 $cm^3$ de tétrahydrofuranne, agite 40 minutes à -70°C, ajoute 13 $cm^3$ de N-diméthylformamide dans 25 $cm^3$ de tétrahydrofuranne et agite 2 heures à -70°C. On verse dans une solution aqueuse de phosphate acide de potassium, extrait à l'éther isopropylique, sèche et évapore le solvant. Après chromatographie sur silice (éluant : hexane-acétate d'éthyle 7-3), on obtient 3,51 g de produit attendu.

Stade B : 5-hydroxyméthyl 2-furanne carbonitrile.

On refroidit à 0°C 0,87 g de l'aldéhyde obtenu au stade A dans 5 $cm^3$ de tétrahydrofuranne et 1 $cm^3$ d'eau et introduit 0,47 g de borohydrure de potassium, agite 5 heures, verse dans une solution aqueuse de phosphate acide de sodium, extrait à l'éther isopropylique, sèche, évapore le solvant et obtient 0,77 g de produit attendu.

**Préparation 17: 1-(3-thiényl) 2-propynol.**

On opère comme au stade B de la préparation 2 à partir de 2,5 g de 3-thiophène carboxaldéhyde. On obtient 2,63 g de produit attendu.

**Préparation 18: 1-(2-thiényl) 2-propynol.**

On opère comme au stade B de la préparation 2 à partir de 4 g de 2-thiophène carboxaldéhyde. On obtient 3,92 g de produit attendu.

**Préparation 19: 1-[5-(difluorométhyl) 2-thiényl] 2-propynol.**

Stade A : 2-méthyl 5-difluorométhyl thiophène.

On opère comme au stade B de la préparation 1 à partir de 10 $cm^3$ de 5-méthyl 2-thiophène carboxaldéhyde. On obtient 9,45 g de produit attendu.

Stade B : 2-bromométhyl 5-difluorométhyl thiophène.

On opère comme au stade E de la préparation 3 à partir de 3,5 g de produit obtenu au stade A. On obtient 3,3 g de produit attendu.

Stade C : 5-difluorométhyl 2-thiophène carboxaldéhyde.

On opère comme au stade F de la préparation 3 à partir de 7,9 g de produit obtenu comme au stade B. On obtient après chromatographie sur silice (éluant : hexane-acétate d'éthyle 8-2) 3,6 g de produit attendu.

Stade D : 1-[5-(difluorométhyl) 2-thiényl] 2-propynol.

On opère comme à la préparation 2 stade B à partir de 3,6 g de l'aldéhyde obtenu au stade C. On obtient 3 g de produit attendu.

**Préparation 20: (5-difluorométhyl 2-thiophène) méthanol.**

En opérant comme à la préparation 16 stade B à partir de l'aldéhyde obtenu au stade C de la préparation 19 et de borohydrure de sodium au sein d'un mélange éthanol/eau, on a préparé le produit attendu.

**Préparation 21: (5-trifluorométhyl 2-furanne) méthanol.**

En opérant comme à la préparation 16 stade B à partir de l'aldéhyde obtenu au stade D de la préparation 6 et du borohydrure de sodium, au sein d'un mélange éthanol/eau, on a préparé le produit attendu.

**Préparation 22: 1-[3-chloro 5-(difluorométhyl) 2-thiényl] 2-propynol.**

Stade A : 4,5-dichloro 2-thiophène carboxaldéhyde.

On refroidit à -60°C 11,1 g de diisopropylamine dans 100 $cm^3$ de tétrahydrofuranne, ajoute en 10 minutes 49 $cm^3$ de n-butyllithium, agite pendant 30 minutes, ajoute à -60°C 10 g de 2,3-dichlorothiophène, agite 45 minutes puis ajoute 6 $cm^3$ de diméthylformamide et 10 $cm^3$ de tétrahydrofuranne. On agite 1 heure et demie, laisse revenir à température ambiante, verse dans une solution aqueuse saturée en phosphate acide de sodium et extrait à l'éther isopropylique. On évapore le solvant et obtient, après chromatographie sur silice (éluant : hexane-acétate d'éthyle 95-5), 10,16 de produit attendu.

Stade B : 2,3-dichloro 5-difluorométhyl thiophène.

On opère comme au stade B de la préparation 1 à partir de 7 g d'aldéhyde préparé au stade A et 7,6 $cm^3$ de DAST. On obtient 5,88 g de produit attendu.

Stade C : 3-chloro 5-difluorométhyl 2-thiophène carboxaldéhyde.

On refroidit à -60°C 2,47 g de produit obtenu au stade B dans 20 $cm^3$ de tétrahydrofuranne et ajoute 9,2 $cm^3$ de n-butyllithium, agite 2 heures, ajoute 1,4 $cm^3$ de diméthylformamide et 3 $cm^3$ de tétrahydrofuranne, agite 30 minutes à -60°C, laisse revenir à température ambiante et poursuit la synthèse comme indiqué au stade A. On obtient 0,9 g de produit attendu.

Stade D : 1-[3-chloro 5-(difluorométhyl) 2-thiényl] 2-propynol.

On opère comme au stade B de la préparation 2 à partir de 0,64 g de produit obtenu au stade C ci-dessus et 8,5 $cm^3$ de bromure d'éthynyl magnésium. On obtient 0,67 g de produit attendu.

**PREPARATION 23: Acide [1R-[1alpha, 3alpha(E,Z)]] 2,2-diméthyl 3-(2,2-difluorocyclopropylidène) méthyl cyclopropane carboxylique et isomères E et Z correspondants**

STADE A : le [1R-[1alpha, 3alpha(R)]] 2,2-diméthyl 3-(1-acétoxy propynyl) cyclopropane carboxylate de (1,1-diméthyl) éthyle.

On introduit à 0°C, en 15 minutes, 10,8 $cm^3$ d'anhydride acétique dans une solution renfermant 8,5 g de [1R-[1alpha, 3alpha(R)]] 2,2-diméthyl 3-(1-hydroxy propynyl) cyclopropane carboxylate de (1,1-diméthyl) éthyle obtenu comme indiqué dans le brevet européen n° 0 105 006 et 25 $cm^3$ de pyridine. On maintient le mélange réactionnel sous agitation pendant 6 heures, le verse sur une solution aqueuse de phosphate acide de sodium, et extrait à l'éther isopropylique. On sèche et amène à sec sous pression réduite. On chromatographie le produit obtenu sur silice en éluant avec le mélange hexane-éther isopropylique (7-3). On obtient 9,44 g de produit de Rf = 0,4. F = 67,2°C.

STADE B : [1R-[1alpha, 3alpha(R)]] 2,2-diméthyl 3-[1-acétoxy 1-(2,2-difluorocyclopropényl) méthyl] cyclopropane carboxylate de (1,1-diméthyl) éthyle

On introduit à 160°C, en 2 H 45 une solution renfermant 61,18 g de chlorodifluoroacétate de sodium et 230 $cm^3$ de diglyme, dans une solution renfermant 10,7 g de produit obtenu au stade A, et 50 $cm^3$ de diglyme. On maintient sous agitation pendant 1 heure, refroidit à 20°C, verse sur une solution aqueuse de phosphate acide de sodium, et extrait à l'éther. On sèche et amène à sec. On élimine le diglyme, en reprenant le produit au pentane. On lave à l'eau, sèche et amène à sec. On chromatographie le produit sur silice en éluant avec le mélange hexane-éther isopropylique (85-15). On obtient 8,57 g de produit recherché de Rf = 0,2.

STADE C : [1R-[1alpha, 3alpha(E-Z)]] 2,2-diméthyl 3-[(2,2-difluorocyclopropylidène) méthyl] cyclopropane carboxylate de (1,1-diméthyl) éthyle, isomère E correspondant, isomère Z correspondant.

On ajoute à la température ambiante 26,6 g d'hydrure de triphénylphosphine de cuivre hexamère $[\Phi_3PCuH]_6$ dans une solution renfermant 8,57 g de produit préparé au stade B et 170 $cm^3$ de toluène. On maintient le mélange réactionnel sous agitation pendant 1 heure. On ajoute 80 $cm^3$ de pentane et 80 $cm^3$ d'éther éthylique. On agite pendant 2 heures, ajoute du clarcel agite à nouveau, filtre et amène à sec. On chromatographie le produit brut obtenu en éluant avec le mélange hexane-chlorure de méthylène (95-5). Après évaporation, sous pression réduite, on obtient :
720 mg de mélange E+Z
2,83 g d'isomère Z, Rf = 0,15 (hexane-chlorure de méthylène 95-5)
2,97 g d'isomère E, Rf = 0,1 ; F = 33,8°C.

STADE D : Acide [1R-[1alpha, 3alpha(Z)]] 2,2-diméthyl 3-[(2,2-difluorocyclopropylidène) méthyl] cyclopropane carboxylique

On porte au reflux pendant 1 h 45 mn, une solution renfermant 2,83 g de produit isomère Z obtenu au stade C, 28 $cm^3$ de toluène et 180 mg d'acide paratoluènesulfonique. On refroidit le mélange réactionnel à 20°C, verse sur de l'eau glacée, extrait à l'éther isopropylique, sèche et amène à sec sous pression réduite. On chromatographie le produit obtenu en éluant avec le mélange hexane-chlorure de méthylène-acétone (70-15-15). On évapore sous pression réduite et recueille 550 mg de produit recherché. Rf = 0,15 (hexane-chlorure de méthylène-acétone 70-15-15).

**Acide [1R-[1alpha, 3alpha(E)]] 2,2-diméthyl 3-[(2,2-difluorocyclopropylidène) méthyl] cyclopropane carboxylique**

En opérant comme pour la préparation de l'acide Z à partir de 2,97 g de l'ester de départ E, on a obtenu 1,88 g du produit recherché. Rf = 0,15 (hexane-chlorure de méthylèneacétone 70-15-15) F = 78,9°C.

**Acide [1R-[1alpha, 3alpha(E+Z)]] 2,2-diméthyl 3-[(2,2-difluorocyclopropylidène) méthyl] cyclopropane carboxylique**

En opérant comme pour la préparation de l'acide Z à partir de 720 mg de l'ester E+Z préparé précédemment, on a obtenu 320 mg du produit recherché. Rf = 0,15 (hexane-chlorure de méthylène-acétone 70-15-15).

**EXEMPLE 47: Préparation d'un concentré soluble**

On effectue un mélange homogène de :

| | |
|---|---|
| Produit de l'exemple 1 | : 0,25 g |
| Butoxyde pipéronyle | : 1,00 g |
| Tween 80 | : 0,25 g |
| Topanol A | : 0,1 g |
| Eau | : 98,4 g |

**EXEMPLE 48: Préparation d'un concentré émulsifiable**

On mélange intimement :

| | |
|---|---|
| Produit de l'exemple 2 | : 0,015 g |
| Butoxyde de pipéronyle | : 0,5 g |
| Topanol A | : 0,1 g |
| Tween 80 | : 3,5 g |
| Xylène | : 95,885 g |

**EXEMPLE 49: Préparation d'un concentré émulsifiable**

On effectue un mélange homogène de :

| | |
|---|---|
| Produit de l'exemple 1 | : 1,5 g |
| Tween 80 | : 20,00 g |

| | |
|---|---|
| Topanol A | : 0,1 g |
| Xylène | : 78,4 g |

**EXEMPLE 50: Préparation de granulés**

On a préparé des granulés contenant de 0,1 % à 5 % de substances actives.

**ETUDE BIOLOGIQUE**

A - Activité sur Diabrotica

Les insectes tests sont des larves de dernier stade de Diabrotica.

On traite une rondelle de papier filtre de 9 cm de diamètre, déposée au fond d'une boîte de Pétri, à l'aide de 1 $cm^3$ de solution acétonique du produit à tester. Après séchage on dépose 15 larves par dose et on effectue le contrôle de mortalité 24 heures après le traitement.

Dès la dose de 0,5 ppm les produits de l'invention présentent une bonne activité, notamment les produits des exemples 1, 5, 7, 9, 10, 12, 18, 19, 26, 27, 28, 30 et 37 et plus particulièrement le produit de l'exemple 2.

B - Activité sur Blatella

**Etude de l'activité par contact tarsal sur blatte germanique.**

Les insectes testés sont des mâles de blatte germanique (Blatella germanica). On opère par dépôt d'une solution acétonique de concentration déterminée sur le fond d'une boîte de Petri de 20 cm de diamètre. Après séchage, on laisse séjourner 20 mâles de blattes par concentration durant 1 heure puis on transfère les insectes sur milieu sain et on contrôle leur mortalité à 24 h, 48 h, 3 et 5 jours.

On détermine ainsi le "KD". Le résultat est exprimé en pourcentage de blattes "KD" en 5 minutes, après traitement par le produit considéré à la dose de 500 mg/l.

Le résultat obtenu est de 100% pour les produits des exemples 5, 6, 9, 13, 19, 23, 24, 25, 29, 31, 33, 35, 36, 40, 42 et 45.

**Revendications**

**1)** Sous toutes leurs formes stéréoisomères possibles ainsi que les mélanges de ces stéréoisomères, les composés de formule (I) :

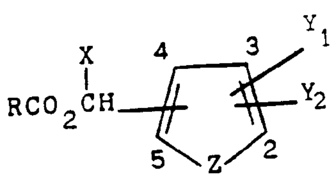

(I)

dans lesquels,

- le radical

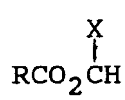

  est en position 2 ou 3,
- X représente un atome d'hydrogène, un radical alkyle linéaire ou ramifié renfermant jusqu'à 8 atomes de carbone, saturé ou insaturé, ou un radical C≡N,
- $Y_1$ et $Y_2$, identiques ou différents en position 2, 3, 4 ou 5 représentent un atome d'hydrogène, un atome d'halogène, un radical

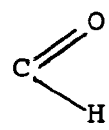 ,

$NO_2$, $NH_2$ ou C≡N, un radical alkyle ou O-alkyle, ou

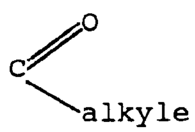 ou 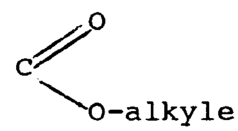

linéaire, ramifié ou cyclique, saturé ou insaturé renfermant jusqu'à 8 atomes de carbone éventuellement substitué par un ou plusieurs atomes d'halogène, un radical $-(CH_2)_nOH$, représentant un nombre entier égal à 0, 1, 2, 3 ou 4 dont le radical OH peut être éventuellement éthérifié ou estérifié, ou un radical

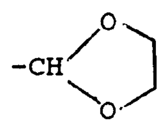

- Z représente un atome d'oxygène ou de soufre,
  et ou bien R représente un radical :

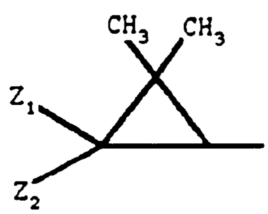

dans lequel :

- $Z_1$ et $Z_2$ représentent chacun un radical méthyle,
- ou $Z_1$ représente un atome d'hydrogène et
- soit $Z_2$ représente un radical :

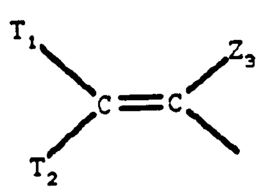

dans lequel $Z_3$ représente un atome d'hydrogène ou d'halogène et $T_1$ et $T_2$, identiques ou différents, représentent un atome d'hydrogène, un atome d'halogène, un radical alkyloxy ou alkyle renfermant de 1 à 8 atomes de carbone éventuellement substitués par des halogènes, un radical mono-, di- ou trifluorométhyle ou cyano ou un noyau phényle éventuellement substitué par un halogène, ou $T_1$ et $T_2$ forment ensemble un radical cycloalkyle renfermant de 3 à 6 atomes de carbone ou un radical :

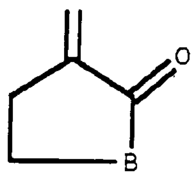

dans lequel B représente un atome d'oxygène ou de soufre ;

- soit $Z_2$ représente un radical :

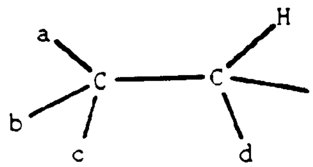

dans lequel a, b, c et d, identiques ou différents représentent chacun un atome d'halogène,

- soit $Z_2$ représente un radical :

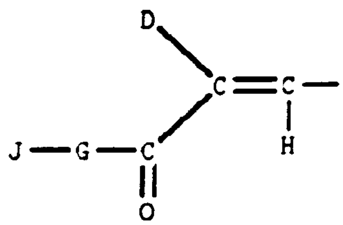

dans lequel D représente un atome d'hydrogène ou d'halogène, un radical alkyloxy renfermant de 1 à 8 atomes de carbone, G représente un atome d'oxygène ou de soufre et J représente un radical alkyle linéaire, ramifié ou cyclique, saturé ou insaturé, renfermant de 1 à 8 atomes de carbone, éventuellement substitué par un ou plusieurs groupements fonctionnels, identiques ou différents, ou bien un groupement aryle renfermant de 6 à 14 atomes de carbone, éventuellement substitué par un ou plusieurs groupements fonctionnels identiques ou différents, ou bien un radical hétérocyclique éventuellement substitué par un ou plusieurs groupements fonctionnels, identiques ou différents,

ou bien R représente un radical :

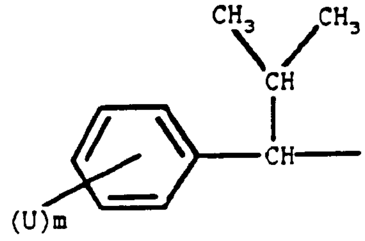

dans lequel U, en position quelconque sur le noyau benzénique, représente un atome d'halogène, un radical alkyle renfermant de 1 à 8 atomes de carbone ou un radical alcoxy renfermant de 1 à 8 atomes de carbone, m représentant le nombre 0, 1 ou 2 et quand m est 2, les substituants U peuvent être identiques ou différents.

**2)** Les composés de formule (I) tels que définis à la revendication 1 dans lesquels Z représente un atome d'oxygène.

**3)** Les composés de formule (I) tels que définis à la revendication 1 ou 2 dans lesquels les radicaux $Y_1$ et $Y_2$ représentent un atome d'hydrogène.

**4)** Les composés de formule (I) tels que définis à la revendication 3, caractérisé en ce que celui des radicaux $Y_1$ ou $Y_2$ qui ne représente pas un atome d'hydrogène est en position 4 ou 5.

**5)** Les composés de formule (I) tels que définis à l'une quelconque des revendications 1 à 4 dans lesquels le radical

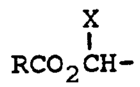

est en position 2.

**6)** Les composés de formule (I) tels que définis à l'une quelconque des revendications 1 à 5 dans lesquels $Y_1$ ou $Y_2$ représente un radical alkyle renfermant jusqu'à 4 atomes de carbone éventuellement substitué par un ou plusieurs atomes de fluor.

**7)** Les composés de formule (I) tels que définis à la revendication 6 dans lesquels $Y_1$ ou $Y_2$ représente un radical $CHF_2$.

**8)** Les composés de formule (I) tels que définis à la revendication 6 dans lesquels $Y_1$ ou $Y_2$ représente un radical $CF_3$.

**9)** Les composés de formule (I) tels que définis à l'une quelconque des revendications 1 à 5 dans lesquels l'un des radicaux $Y_1$ ou $Y_2$ représente un radical $NO_2$, $-C{\equiv}CH$ ou $-CH_2-C{\equiv}CH$.

**10)** Les composés de formule (I) tels que définis à l'une quelconque des revendications 1 à 9 dans lesquels X représente un atome d'hydrogène.

**11)** Les composés de formule (I) tels que définis à l'une quelconque des revendications 1 à 9 dans lesquels X représente un radical éthynyle.

**12)** Les composés de formule (I) tels que définis à l'une quelconque des revendications 1 à 11 dans lesquels R représente le reste d'un acide dérivé de l'acide cyclopropane carboxylique dans lequel la copule cyclopropanique est de structure 1 R cis.

**13)** Les composés de formule (I) tels que définis à l'une quelconque des revendications 1 à 12 dans lesquels R représente le radical :

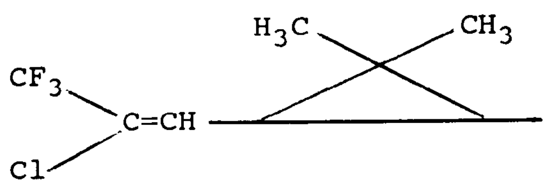

sous toutes ses formes stéréoisomères possibles, ainsi que leurs mélanges.

**14)** Les composés de formule (I) tels que définis à la revendication 1 dont les noms suivent :

- le [1R-[1alpha,3alpha(Z)]] 2,2-diméthyl 3-(3,3,3-trifluoro 2-chloropropényl) cyclopropane carboxylate de [5-(difluorométhyl) 2-furyl] méthyle ;
- le [1R-[1alpha,3alpha(Z)]] 2,2-diméthyl 3-(2-fluoro 3-méthoxy 3-oxo 1-propényl) cyclopropane carboxylate de 1-[5-(difluorométhyl) 2-furyl] méthyle ;
- le [1R-[1alpha,3alpha(Z)]] 2,2-diméthyl 3-(2,2-dichloro éthényl) cyclopropane carboxylate de 1-[5-(difluorométhyl) 2-furyl] méthyle ;
- le [1R-[1alpha,3alpha(Z)]] 2,2-diméthyl 3-(2,2-difluoro cyclopropylidène) méthyl cyclopropane carboxylate de 1-[5-(difluorométhyl) 2-furyl] méthyle ;
- le [1R-[1alpha,3alpha(Z)]] 2,2-diméthyl 3-[3-[(1-trifluorométhyl) 2,2,2-trifluoroéthoxy] 3-oxo 1-propényl] cyclopropane carboxylate de 1-[5-(difluorométhyl) 2-furyl] 2-propynyle ;
- le [1R-[1alpha,3alpha(Z)]] 2,2-diméthyl 3-[3,3,3-trifluoro 2-chloropropényl] cyclopropane carboxylate de (S) 1-[5-(difluorométhyl) 2-furyl] 2-propynyle et [1R-[1alpha 3alpha (Z)]] 2,2-diméthyl 3-[3,3,3-trifluoro 2-chloropropényl] cyclopropane carboxylate de (R) 1-[5-(difluorométhyl) 2-furyl] 2-propynyle ;
- le [1R-[1alpha,3alpha(Z)]] 2,2-diméthyl 3-[3,3,3-trifluoro 2-chloropropényl] cyclopropane carboxylate de 1-[5-(trifluorométhyl) 2-furyl] 2-propynyle ;
- le [1R-[1alpha,3alpha(Z)]] 2,2-diméthyl 3-(2-fluoro 3-méthoxy 3-oxo 1-propényl) cyclopropane carboxylate de 1-[5-(trifluorométhyl) 2-furyl] 2-propynyle ;
- le [1R-[1alpha,3alpha(Z)]] 2,2-diméthyl 3-[3,3,3-trifluoro 2-chloropropényl] cyclopropane carboxylate de (5-éthynyl 2-furyl) méthyle ;
- le [1R-[1alpha,3alpha(Z)]] 2,2-diméthyl 3-[3,3,3-trifluoro 2-chloropropényl] cyclopropane carboxylate de 1-(5-éthynyl 2-furyl) 2-propynyle ;
- le [1R-[1alpha,3alpha(Z)]] 2,2-diméthyl 3-[3,3,3-trifluoro 2-chloropropényl] cyclopropane carboxylate de

[2-(difluorométhyl) 4-furyl] méthyle ;
- le [1R-[1alpha,3alpha(Z)]] 2,2-diméthyl 3-[3,3,3-trifluoro 2-chloropropényl] cyclopropane carboxylate de 1-[2-(difluorométhyl) 4-furyl] 2-propynyle ;
- le [1R-[1alpha,3alpha(Z)]] 2,2-diméthyl 3-[3,3,3-trifluoro 2-chloropropényl] cyclopropane carboxylate de [5-(trifluorométhyl) 2-furyl] méthyle.

**15)** Le composé de formule (I) tel que défini à la revendication 1 dont le nom suit :
- le [1R-[1alpha,3alpha (Z)] 2,2-diméthyl-3-(3,3,3-trifluoro 2-chloro propényl) cyclopropane carboxylate de [5-difluorométhyl 2-furyl] 2-propynyle.

**16)** Procédé de préparation des composés de formule (I) tels que définis à l'une quelconque des revendications 1 à 15 caractérisé en ce que l'on soumet un acide de formule (II) :

$$RCO_2H$$

dans laquelle R est défini comme précédemment ou un dérivé fonctionnel de cet acide à l'action d'un alcool de formule (III) :

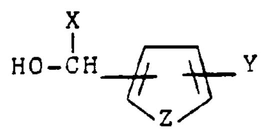

(III)

dans laquelle X, Y et Z sont définis comme précédemment ou d'un dérivé fonctionnel de cet alcool pour obtenir le composé de formule (I) correspondant.

**17)** A titre de produits chimiques nouveaux, les composés de formule (III) tels que définis à la revendication 16.

**18)** A titre de produits chimiques nouveaux, les composés de formule (III) tels que définis à la revendication 16 dans lesquels Y représente un radical $CHF_2$, $CF_3$, $NO_2$, $-C\equiv CH$, $CH_2-C\equiv CH$.

**19)** A titre de produits chimiques nouveaux, les composés de formule (III) tels que définis à la revendication 14 dont les noms suivent :
- le (5-difluorométhyl 2-furan) méthanol ;
- le (5-difluorométhyl alpha-éthynyl 2-furan) méthanol ;
- le [4-trifluorométhyl 2-furyl] 2-propynol ;
- le 2-trifluorométhyl 3-furan méthanol ;
- le 2-trifluorométhyl 5-(2-propynyl) 3-furanne méthanol ;
- le 1-(5-trifluorométhyl 2-furyl) 2-propynol ;
- le 1-(2-furyl) 2-propynol ;
- le (5-méthoxyméthyl 2-furanne) méthanol ;
- le 1-(5-méthoxyméthyl 2-furyl) 2-propynol ;
- le 5-éthynyl 2-furanne méthanol ;
- le 1-(5-éthynyl 2-furyl) 2-propynol ;
- le 5-difluorométhyl 3-furanne méthanol ;
- le 1-(5-difluorométhyl 3-furyl) 2-propynol ;
- le 2-difluorométhyl 3-furanne méthanol ;
- le 1-(5-nitro 2-furyl) 2-propynol ;
- le 5-hydroxyméthyl 2-furanne carbonitrile ;
- le 1-(3-thiényl) 2-propynol ;
- le 1-(2-thiényl) 2-propynol ;
- le 1-[5-(difluorométhyl) 2-thiényl] 2-propynol ;
- le (5-difluorométhyl 2-thiophène) méthanol ;
- le (5-trifluorométhyl 2-furanne) méthanol ;
- le 1-[3-chloro 5-(difluorométhyl) 2-thiényl] 2-propynol.

**20)** Application des composés de formule (I) tels que définis à l'une quelconque des revendications 1 à 15, à la lutte contre les parasites des végétaux et les parasites des locaux.

**21)** Les compositions destinées à la lutte contre les parasites des végétaux, les parasites des locaux et les parasites des animaux à sang chaud, caractérisées en ce qu'elles renferment comme principe actif au moins un composé défini à l'une quelconque des revendications 1 à 15.

**22)** Les compositions insecticides, caractérisées en ce qu'elles renferment comme principe actif au moins un composé défini à l'une quelconque des revendications 1 à 15.

**23)** Les compositions insecticides définies à la revendication 22 caractérisées en ce qu'elles sont destinées

à la lutte contre DIABROTICA et les autres parasites du sol.

**24)** Les compositions acaricides, caractérisées en ce qu'elles renferment comme principe actif au moins un composé défini à l'une quelconque des revendications 1 à 15.

**25)** Les compositions définies à l'une quelconque des revendications 21 à 24 caractérisées en ce qu'elles renferment comme principe actif au moins un composé défini à la revendication 14.

**26)** Les compositions définies à l'une quelconque des revendications 21 à 23 caractérisées en ce qu'elles renferment comme principe actif au moins un composé défini à la revendication 15.

**27)** Associations douées d'activité insecticide, acaricide ou nématicide, caractérisées en ce qu'elles contiennent comme matière active, d'une part, un au moins des composés de formule générale (I) et d'autre part, un au moins des esters pyréthrinoïdes choisis dans le groupe constitué par les esters d'alléthrolone, d'alcool 3,4,5,6-tétrahydrophtalimido méthylique, d'alcool 5-benzyl 3-furyl méthylique, d'alcool 3-phénoxy benzylique et d'alcool alpha-cyano 3-phénoxy benzylique des acides chrysanthémiques, par les esters d'alcool 5-benzyl 3-furyl méthylique des acides 2,2-diméthyl 3-(2-oxo 3-tétrahydrothiophénylidène méthyl) cyclopropanecarboxyliques, par les esters d'alcool 3-phénoxy benzylique et d'alcool alphacyano 3-phénoxy benzylique des acides 2,2-diméthyl 3-(2,2-dichlorovinyl) cyclopropanecarboxyliques, par les esters d'alcool alpha-cyano 3-phénoxy benzylique d'acides 2,2-diméthyl 3-(2,2-dibromovinyl) cyclopropanecarboxyliques, par les esters d'alcool 3-phénoxy benzylique des acides 2-parachlorophényl 2-isopropyl acétiques, par les esters d'alléthrolone, d'alcool 3,4,5,6-tétrahydrophtalimidométhylique, d'alcool 5-benzyl 3-furyl méthylique, d'alcool 3-phénoxy benzylique et d'alcool alpha-cyano 3-phénoxy benzylique des acides 2,2-diméthyl 3-(1,2,2,2-tétrahaloéthyl) cyclopropanecarboxyliques, dans lesquels "halo" représente un atome de fluor, de chlore ou de brome, étant entendu que les composés (I) peuvent exister sous toutes leurs formes stéréoisomères possibles de même que les copules acides et alcools des esters pyréthrinoïdes ci-dessus.

Office européen des brevets

# RAPPORT DE RECHERCHE EUROPEENNE

Numero de la demande

EP 93 40 0289

| DOCUMENTS CONSIDERES COMME PERTINENTS | | | |
|---|---|---|---|
| Catégorie | Citation du document avec indication, en cas de besoin, des parties pertinentes | Revendication concernée | CLASSEMENT DE LA DEMANDE (Int. Cl.5) |
| X | FR-A-2 442 826 (MONTEDISON S.p.A.)<br>* Page 3, ligne 1 - page 4, ligne 30; revendications 1,2,50 *<br>--- | 1-22 | C 07 D 307/42<br>A 01 N 53/00 |
| X | GB-A-1 219 830 (YOSHITOMI PHARMACEUTICAL INDUSTRIES LTD)<br>* Le document en entier *<br>--- | 1-22 | |
| X | EP-A-0 009 709 (BAYER AG)<br>* Abrégé; revendications *<br>--- | 1-22 | |
| X | FR-A-1 541 893 (SUMITOMO CHEMICAL CO., LTD)<br>* Le document en entier *<br>--- | 1-22 | |
| X | FR-A-2 016 577 (BASF AG)<br>* Revendications; exemple 20 *<br>--- | 1-22 | |
| X | CHEMICAL ABSTRACTS, vol. 86, no. 5, 31 janvier 1977, page 349, colonne 1, abrégé no. 29608r, Columbus, Ohio, US; & JP-A-51 004 993 (YOSHITOMI PHARMACEUTICAL INDUSTRIES, LTD) 16-02-1976<br>* Abrégé *<br>--- | 1-22 | DOMAINES TECHNIQUES RECHERCHES (Int. Cl.5)<br>C 07 D |
| X | DE-A-2 166 237 (SUMITOMO CHEMICAL CO., LTD)<br>* Le document en entier *<br>--- | 16-17 | |
| A | EP-A-0 105 006 (ROUSSEL-UCLAF)<br>* Abrégé; revendications; page 2, lignes 30-35 *<br>--- -/- | 1-22 | |

Le présent rapport a été établi pour toutes les revendications

| Lieu de la recherche | Date d'achèvement de la recherche | Examinateur |
|---|---|---|
| LA HAYE | 17-05-1993 | LUYTEN H W |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)

Page 2

Office européen des brevets

# RAPPORT DE RECHERCHE EUROPEENNE

Numero de la demande

EP 93 40 0289

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| A | FR-A-2 439 780 (SUMITOMO CHEMICAL CO., LTD)<br>* Revendications *<br>----- | 1-22 | |
| | | | DOMAINES TECHNIQUES RECHERCHES (Int. Cl.5) |
| | | | |

Le présent rapport a été établi pour toutes les revendications

| Lieu de la recherche | Date d'achèvement de la recherche | Examinateur |
|---|---|---|
| LA HAYE | 17-05-1993 | LUYTEN H W |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
& : membre de la même famille, document correspondant

EPO FORM 1503 03.82 (P0402)